(19) Europäisches Patentamt
European Patent Office
Office européen des brevets

(11) EP 0 828 487 B1

(12) EUROPÄISCHE PATENTSCHRIFT

(45) Veröffentlichungstag und Bekanntmachung des Hinweises auf die Patenterteilung:
**26.11.2003 Patentblatt 2003/48**

(21) Anmeldenummer: **96919789.6**

(22) Anmeldetag: **20.05.1996**

(51) Int Cl.[7]: **A61K 31/415**, A61K 31/505, A61K 31/53, A61K 31/35, A01N 43/52 // (A61K31/415, 31:35), (A61K31/505, 31:415), (A61K31/53, 31:415, A61P33:00)

(86) Internationale Anmeldenummer:
**PCT/EP96/02164**

(87) Internationale Veröffentlichungsnummer:
**WO 96/038140 (05.12.1996 Gazette 1996/53)**

# (54) MITTEL GEGEN PARASITÄRE PROTOZOEN

AGENTS FOR USE AGAINST PARASITIC PROTOZOA

AGENTS POUR LUTTER CONTRE LES PROTOZOAIRES PARASITES

(84) Benannte Vertragsstaaten:
**AT BE CH DE DK ES FI FR GB GR IE IT LI NL SE**

(30) Priorität: **31.05.1995 DE 19519821**

(43) Veröffentlichungstag der Anmeldung:
**18.03.1998 Patentblatt 1998/12**

(73) Patentinhaber: **BAYER AG**
**51368 Leverkusen (DE)**

(72) Erfinder:
- **ASSMANN, Lutz**
  **D-23701 Eutin (DE)**
- **BAASNER, Bernd**
  **D-51467 Bergisch Gladbach (DE)**
- **HABERKORN, Axel**
  **D-42117 Wuppertal (DE)**
- **LIEB, Folker**
  **D-51375 Leverkusen (DE)**
- **LUNKENHEIMER, Winfried**
  **D-42115 Wuppertal (DE)**
- **LUI, Norbert**
  **D-51061 Köln (DE)**

(56) Entgegenhaltungen:
**DE-A- 4 237 617** **US-A- 5 331 003**

Bemerkungen:
Die Akte enthält technische Angaben, die nach dem Eingang der Anmeldung eingereicht wurden und die nicht in dieser Patentschrift enthalten sind.

EP 0 828 487 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft Mischungen von substituierten Benzimidazolen mit einem Polyetherantibiotikum oder einem synthetisch hergestellten Coccidiosemittel als Mittel zur Bekämpfung parasitärer Protozoen und insbesondere Coccidien sowie Fisch- und Insektenparasiten

Substituierte Benzimidazole und ihre Verwendung als Insektizide, Fungizide und Herbizide sind bereits bekannt geworden (EP-OS 87 375, 152 360, 181 826, 239 508, 260 744, 266 984, US-P 3 418 318, 3 472 865, 3 576 818, 3 728 994).

Halogenierte Benzimidazole und ihre Wirkung als Anthelmintika, Coccidiostatika und Pestizide sind bekannt geworden (DE-OS 2 047 369, DE-OS 4 237 617). Mischungen von nitrosubstituierten Benzimidazolen und Polyetherantibiotika sind als Coccidiosemittel bekannt geworden (US-P 5 331 003) In allen Fällen befriedigt ihre Wirkung noch nicht.

Coccidiose ist eine Erkrankung, die durch einzellige Parasiten (Protozoen) hervorgerufen wird. Insbesondere bei der Geflugelaufzucht kann sie große Verluste hervorrufen. Um diese zu vermeiden, werden die Bestande prophylaktisch mit Coccidiosemittel behandelt. Durch die Entwicklung von Resistenzen gegen die eingesetzten Mittel kommt es schon nach relativ wenigen Jahren zu ernsthaften Problemen. Durch den Einsatz chemisch vollig neuer Coccidiosemittel, insbesondere Kombinationen, ist es andererseits moglich, auch polyresistente Parasitenstämme zu kontrollieren.

Gegenstand der vorliegenden Erfindung sind Mischungen von substituierten Benzimidazolen der Formel (I)

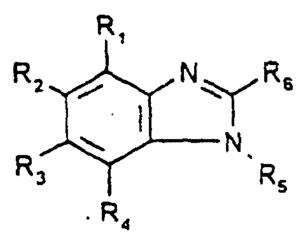

(I)

in welcher

$R_1$, $R_2$, $R_3$ und $R_4$ unabhängig voneinander jeweils für Wasserstoff, Halogen, für jeweils gegebenenfalls substituiertes Alkyl, Alkoxy, Alkylthio, Alkylsulfinyl, Alkylsulfonyl, für gegebenenfalls substituiertes ankondensiertes Dioxyalkylen stehen, wobei jedoch mindestens einer der Substituenten $R_1$, $R_2$, $R_3$ und $R_4$ verschieden von Wasserstoff und Halogen ist,

$R_5$ für Wasserstoff, für Alkyl steht, das ein- oder mehrfach, gleich oder verschieden substituiert ist durch OH, CN, $NH_2$, Alkyl, Cycloalkyl, Alkenyl, Alkinyl, Alkoxy, Halogenalkoxy, Alkylthio, Halogenalkylthio, Alkenoxy, Alkinoxy, Aminocarbonyl, gegebenenfalls substituiertes Alkylcarbonyl, gegebenenfalls substituiertes (Het-)arylcarbonyl, gegebenenfalls substituiertes Alkoxycarbonyl (AlkO-CO-), gegebenenfalls substituiertes Alkoxycarbonyloxy (AlkOCOO-), Aminosulfonyl ($SO_2NH_2$), gegebenenfalls substituiertes Mono- oder Dialkylaminosulfonyl, acyliertes Amino (AlkCON($R_7$)- oder AlkOCON($R_7$)-), wobei $R_7$ gleich Wasserstoff, Alkyl oder Cycloalkyl ist, gegebenenfalls substituiertes Alkylsulfonylamino (Alkyl$SO_2$NH-), oder Alkylsulfonyl-N-alkylamino (Aryl$SO_2$NAlkyl-), gegebenenfalls substituiertes Arylsulfonylamino (Aryl$SO_2$NH-) oder Arylsulfonyl-N-alkylamino (Aryl$SO_2$NAlk-), gegebenenfalls substituiertes Dialkylamino, ferner steht $R_5$ für gegebenenfalls substituiertes Alkoxycarbonyl, gegebenenfalls substituiertes (Het-)Aryloxycarbonyl, Alkylsulfonyl, Alkenylsulfonyl, (Het-)Arylsulfonyl, oder $-SO_2NR_8R_9$, $-CONR_8R_9$ bzw - P(O)$(NR_8R_9)_2$, wobei $R_8$ und $R_9$ fur H oder Alkyl stehen, das gegebenenfalls durch einen oder mehrere Reste substituiert ist.

$R_6$ fur Fluoralkyl steht,

mit einer oder mehreren der folgenden Verbindungen
Polyetherantibiotika wie Maduramycin, Lasalocid, Monensin, Narasin, Salinomycin oder synthetische Coccidiosemittel ausgewählt aus

| | |
|---|---|
| 1(-(4-Amino-2-n-propyl-5-pyrimidinylmethyl)-2-picolinium-chlorid | Amprolium |
| 1(-(4-Amino-2-n-propyl-5-pyrimidinylmethyl)-2-picolinium-chlorid + Sulfaquinoxalin | Amprolium + Sulfaquinoxalin |
| 1(-(4-Amino-2-n-propyl-5-pyrimidinylmethyl)-2-picolinium-chlorid + Sulfaquinoxalin + Ethopabate | Amprolium + Sulfaquinoxatin + Ethopabate |
| 4,4-Dinitrocarbanilid + 2-Hydroxy-4,6-dimethylpyrimidin | Nicarbazin |
| 3,5-Dichloro-2,6-dimethyl-4-pyridinol | Clopidol |
| 3,5-Dichloro-2,6-dimethyl-4-pyridinol + methyl-7-benzyloxy-6-butyl-1,4-dihydro-4-oxylquinolin-3-carboxylate | Clopidol + Methylbenzoquate |
| Ethyl 6-n-decyloxy-7-ethoxy-4-hydroxychinolin-3-carboxylat | Decoquinate |
| 9-(2-Chloro-6-fluorophenylmethyl)-9H-purin-6-amin | Arprinocid |
| (±)-2,6-Dichloro-alpha-(4-chlorophenyl)-4-(4,5-dihydro-3,5-dioxo-1,2,4-triazin-2(3H)-yl)-benzeneacetonitril | Benzeneacetonitril, Diclazuril |
| 1-[3-Methyl-4-(4'-trifluoromethylthiophenoxy)-phenyl]-3-methyl-1,3,5-triazin-2,4,6(1H,3H,5H)-trion | Toltrazuril |
| 4,4-Dinitrocarbanilid + 2-Hydroxy-4,6-dimethylpyrimidin [= Nicarbazin] | Robenidin |
| 7-Bromo-6-chloro-febrifugin | Halofuginon |
| 3,5-Dinitro-o-toluamid | Zoalen |

als Mittel zur Bekampfung parasitarer Protozoen, insbesondere Coccidien bei Tieren

Die erfindungsgemaß verwendbaren Benzimidazole sind bekannt. Sie sind durch die Formel (I) allgemein definiert. Bevorzugt sind Verbindungen der Formel (I), bei welchen

$R_1$, $R_2$, $R_3$ und $R_4$ unabhangig voneinander jeweils fur Wasserstoff, Fluor, Chlor, Brom, Iod, fur jeweils geradkettiges oder verzweigtes Alkyl, Alkoxy, Alkylthio, Alkylsulfinyl, Alkylsulfonyl mit jeweils 1 bis 8 Kohlenstoffatomen, für Cycloalkyl mit 3 bis 8 Kohlenstoffatomen, fur jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschiedenen Halogenatomen oder fur gegebenenfalls einfach oder mehrfach, gleich oder verschieden durch 1 bis 10 Halogene und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 Halogenatomen zweifach verknüpftes Dioxyalkylen mit 1 bis 5 Kohlenstoffatomen stehen.

wobei jedoch mindestens einer der Substituenten $R_1$, $R_2$, $R_3$ und $R_4$ verschieden von Wasserstoff und Halogen ist,

$R_5$ für Wasserstoff, fur Alkyl mit 1 bis 4 Kohlenstoffatomen steht, das ein- oder mehrfach, gleich oder verschieden substituiert ist durch OH, CN, $NH_2$, Alkyl mit 1 bis 4 Kohlenstoffatomen, Cycloalkyl mit 3 bis 10 Kohlenstoffatomen, Alkenyl mit 2 bis 6 Kohlenstoffatomen, Alkinyl mit 2 bis 6 Kohlenstoffatomen, Alkyloxy mit 1 bis 6 Kohlenstoffatomen, Halogenalkoxy mit 1 bis 6 Kohlenstoffatomen und 1 bis 5 Halogenen, Alkylthio mit 1 bis 6 Kohlenstoffatomen, Halogenalkylthio mit 1 bis 6 Kohlenstoffatomen und 1 bis 5 Halogenen, Alkenyloxy mit 2 bis 6 Kohlenstoffatomen, Alkinyloxy mit 3 bis 6 Kohlenstoffatomen, gegebenenfalls substituiertes Alkylcarbonyl mit 1 bis 6 Kohlenstoffatomen, gegebenenfalls substituiertes Phenyl- oder Hetarylcarbonyl, gegebenenfalls substituiertes Alkoxycarbonyl mit 1 bis 6 Kohlenstoffatomen, gegebenenfalls substituiertes Alkoxycarbonyloxy mit 1 bis 6 Kohlenstoffatomen, Aminosulfonyl ($NH_2SO_2$-), gegebenenfalls substitutertes Mono- oder Dialkylaminosulfonyl mit 1 bis 6 Kohlenstoffatomen, acyliertes Amino (AlkOCON($R_7$)- oder (AlkCON($R_7$)-). wobei $R_7$ gleich Wasserstoff oder Alkyl mit 1 bis 6 Kohlenstoffatomen oder Cycloalkyl mit 3 bis 8 Kohlenstoffatomen ist, mit 1 bis 6 Kohlenstoffatomen, gegebenenfalls substituiertes Alkylsulfonylamino mit 1 bis 6 Kohlenstoffatomen, gegebenenfalls substituiertes Alkylsulfonyl-N-alkylamino mit 1 bis 6 Kohlenstoffatomen, gegebenenfalls substituiertes Phenylsulfonylamino, gegebenenfalls substituiertes Phenylsulfonyl-N-alkylamino mit 1 bis 6 Kohlenstoffatomen, gegebenenfalls substituiertes Dialkylamino mit 1 bis 8 Kohlenstoffatomen, ferner steht $R_5$ für die gegebenenfalls substituierten Reste Alkyloxycarbonyl mit 1 bis 6 Kohlenstoffatomen, Alkylsulfonyl oder Alkenylsulfonyl mit 1 bis 6 Kohlenstoffatomen, die gegebenenfalls substituiert sind mit 1 bis 13 Halogenen, gegebenenfalls substituiertes Phenylsulfonyl, gegebenenfalls substituiertes Heteroarylsulfonyl, Phenoxycarbonyl, oder $-SO_2NR_8R_9$, $-CONR_8R_9$ bzw. $-P(O)(NR_8R_9)_2$, wobei $R_8$ und $R_9$ fur Wasserstoff oder ein Alkyl mit 1 bis 4 Kohlenstoffatomen

steht, die gegebenenfalls durch eine der bei $R_5$ oben genannten Reste substituiert sind,

Als Substituenten der gegebenenfalls substituierten Reste seien die folgenden Substituenten genannt:

Halogen, OH, $NH_2$, Alkylamino mit 1 bis 6 Kohlenstoffatomen, Cyano, Nitro, $CO_2H$, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxyalkyl, Alkoxyalkoxy, Alkanoyl, Alkoxycarbonyl oder Alkoximinoalkyl mit jeweils 1 bis 6 Kohlenstoffatomen in den einzelnen Alkylteilen, gegebenenfalls einfach bis sechsfach, gleich oder verschieden durch Halogen und/oder geradkettiges oder verzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen substituiertes, zweifach verknüpftes Dioxyalkylen mit 1 bis 6 Kohlenstoffatomen oder gegebenenfalls einfach bis fünffach, gleich oder verschieden durch Halogen und/oder geradkettiges oder verzweigtes Alkyl mit 1 bis 4 Kohlenstoffatomen und 1 bis 9 gleichen oder verschiedenen Halogenatomen substituierten Phenyl oder Phenoxy, die ihrerseits die oben genannten Reste tragen können

Als Acylreste der aufgeführten Reste seien genannt die Reste Alkoxycarbonyl mit 1 bis 6 Kohlenstoffatomen, Alkylcarbonyl mit 1 bis 6 Kohlenstoffatomen, Alkoxycarbonyloxy mit 1 bis 6 Kohlenstoffatomen, Alkylsulfonyl mit 1 bis 6 Kohlenstoffatomen, Benzoyl, das gegebenenfalls substituiert ist durch einen der oben aufgefuhrten Reste, Alkenylcarbonyl mit 2 bis 6 Kohlenstoffatomen

$R_6$ fur 1 bis 15 Fluor-$C_1$-$C_7$-alkyl steht

Besonders bevorzugt haben in Verbindungen der Formel (I) die Substituenten folgende Bedeutung.

$R_1$, $R_2$, $R_3$ und $R_4$ unabhängig voneinander jeweils für Wasserstoff, Fluor, Chlor oder Brom, fur jeweils geradkettiges oder verzweigtes Alkyl, Alkoxy, Alkylthio, Alkylsulfinyl, Alkylsulfonyl mit jeweils 1 bis 4 Kohlenstoffatomen, fur Cycloalkyl mit 3 bis 6 Kohlenstoffatomen, für jeweils geradkettiges oder verzweigtes Halogenalkyl, Halogenalkoxy, Halogenalkylthio, Halogenalkylsulfinyl, Halogenalkylsulfonyl mit jeweils 1 bis 4 Kohlenstoffatomen und 1 bis 9 gleichen oder verschiedenen Halogenatomen, insbesondere Fluor- und Chloratome oder für gegebenenfalls einfach oder mehrfach, gleich oder verschieden durch 1 bis 6 Halogene, insbesondere Fluor- und Chloratomen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 6 Halogenatomen zweifach verknüpftes Dioxyalkylen mit 1 bis 3 Kohlenstoffatomen stehen, wobei jedoch mindestens einer der Substituenten $R_1$, $R_2$, $R_3$ und $R_4$ verschieden von Wasserstoff und Halogen ist,

$R_5$ für Wasserstoff, fur Alkyl mit 1 bis 3 Kohlenstoffatomen, insbesondere für Methyl und Ethyl steht, das substituiert ist durch OH, CN, $NH_2$, Alkyl mit 1 bis 4 Kohlenstoffatomen, insbesondere Methyl oder Ethyl, Alkenyl mit 2 bis 4 Kohlenstoffatomen, Alkinyl mit 2 bis 4 Kohlenstoffatomen, Alkyloxy mit 1 bis 4 Kohlenstoffatomen, insbesondere Alkoxy wie Methoxy, Ethoxy, Propoxy, i-Propoxy, Halogenalkoxy mit 1 bis 4 Kohlenstoffatomen und 1 bis 5 Halogenen, insbesondere Trifluormethoxy, Pentafluorethoxy, Fluorpropoxy, Alkylthio mit 1 bis 4 Kohlenstoffatomen, Halogenalkylthio mit 1 bis 4 Kohlenstoffatomen und 1 bis 5 Halogenen, Alkenyloxy mit 2 bis 4 Kohlenstoffatomen, Alkinyloxy mit 3 bis 4 Kohlenstoffatomen, gegebenenfalls substituiertes Alkylcarbonyl mit 1 bis 4 Kohlenstoffatomen, gegebenenfalls substituiertes Phenylcarbonyl, gegebenenfalls substituiertes Alkoxycarbonyl mit 1 bis 4 Kohlenstoffatomen, insbesondere Alkoxycarbonyle wie Methoxycarbonyl, Ethoxycarbonyl, Propoxycarbonyl, i-Propoxycarbonyl, t-Butoxycarbonyl, gegebenenfalls substituiertes Alkoxycarbonyloxy mit 1 bis 4 Kohlenstoffatomen, Aminosulfonyl ($NH_2SO_2$-), gegebenenfalls substituiertes Mono- oder Dialkylaminosulfonyl mit 1 bis 4 Kohlenstoffatomen, acyliertes Amino mit 1 bis 6 Kohlenstoffatomen, wobei $R_7$ gleich Wasserstoff oder Alkyl mit 1 bis 4 Kohlenstoffatomen oder Cycloalkyl mit 3 bis 6 Kohlenstoffatomen ist. gegebenenfalls substituiertes Alkylsulfonylamino mit 1 bis 4 Kohlenstoffatomen, gegebenenfalls substituiertes Alkylsulfonyl-N-alkylamino mit 1 bis 4 Kohlenstoffatomen, gegebenenfalls substituiertes Phenylsulfonylamino, gegebenenfalls substituiertes Phenylsulfonyl-N-alkylamino mit 1 bis 4 Kohlenstoffatomen, gegebenenfalls substituiertes Dialkylamino mit 1 bis 4 Kohlenstoffatomen, ferner steht $R_5$ fur die gegebenenfalls substituierten Reste Alkyloxycarbonyl mit 1 bis 4 Kohlenstoffatomen, Alkylsulfonyl oder Alkenylsulfonyl mit 1 bis 4 Kohlenstoffatomen, die gegebenenfalls substituiert sind mit 1 bis 9 Halogenen, gegebenenfalls substituiertes Phenylsulfonyl, Heteroarylsulfonyl, wobei Heteroaryl für einen 5 bzw. 6-gliedrigen Heterocyclus steht, der gegebenenfalls substituiert ist mit Alkyl mit 1 bis 4 Kohlenstoffatomen oder Halogen oder einem unter $R_5$ aufgeführten Substituenten, Phenoxycarbonyl, oder $SO_2NR_8R_9$, $CONR_8R_9$ bzw. - $P(O)(NR_8R_9)_2$, wobei $R_8$ und $R_9$ fur Wasserstoff oder ein Alkyl mit 1 bis 4 Kohlenstoffatomen steht, die gegebenenfalls durch eine der bei $R_5$ oben genannten Reste substituiert sind.

Als Substituenten der gegebenenfalls substituierten Reste seien die folgenden Substituenten genannt

Halogen, insbesondere Fluor oder Chlor, OH, $NH_2$, Alkylamino mit 1 bis 4 Kohlenstoffatomen, insbesondere Methylamino, Ethylamino, Dimethylamino, Diethylamino, Bis-(trifluormethylamino), Cyano, Nitro, $CO_2H$, jeweils geradkettiges oder verzweigtes Alkyl, Alkoxy, Alkylthio, Alkylsulfinyl oder Alkylsulfonyl mit jeweils 1 bis 4 Kohlenstoffatomen, insbesondere Methyl, Ethyl, Methoxy, Ethoxy oder Methylmercapto, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, insbesondere Trifluormethyl, Pentafluorethyl, Fluorchlorethyl, Trichlorethyl, Trifluormethoxy, Trichlormethoxy, Trifluormethylmercapto, jeweils geradkettiges oder verzweigtes Alkoxyalkyl, Alkoxyalkoxy, insbesondere Methoxyethoxy, Ethoxyethoxy, Ethoxyethyl, Alkanoyl. Alkoxycarbonyl oder Alkoximinoalkyl mit jeweils 1 bis 4 Kohlenstoffatomen in den einzelnen Alkylteilen, insbesondere Acetyl, gegebenenfalls einfach bis funffach, gleich oder verschieden durch Halogen und/oder geradkettiges oder verzweigtes Alkyl mit 1 bis 4 Kohlenstoffatomen und 1 bis 9 gleichen oder verschiedenen Halogenatomen substituiertes Phenyl oder Phenoxy, insbesondere Phenyl oder Phenoxy, die ihrerseits die oben genannten Reste tragen können.

$R_6$ steht fur 1 bis 7 Fluor-$C_1$-$C_3$-alkyl.

Ganz besonders bevorzugt sind Verbindungen der Formel (I), in welcher die Reste folgende Bedeutung haben:

$R_1$, $R_2$, $R_3$ und $R_4$ stehen für Wasserstoff, Halogen, insbesondere Chlor oder Brom, für jeweils geradkettiges oder verzweigtes Alkyl, Alkoxy, Alkylthio, Alkylsulfinyl, Alkylsulfonyl mit jeweils 1 bis 4 Kohlenstoffatomen, insbesondere Methyl, Methoxy, Methylthio, Methylsulfinyl oder Methylsulfonyl, fur jeweils geradkettiges oder verzweigtes Halogenalkyl, Halogenalkoxy, Halogenalkylthio, Halogenalkylsulfinyl, Halogenalkylsulfonyl mit jeweils 1 bis 4 Kohlenstoffatomen und 1 bis 9 gleichen oder verschiedenen Fluor oder Chloratomen, insbesondere für Trifluormethyl, Trifluormethoxy, Trifluormethylthio, Trifluormethylsulfinyl, Trifluormethylsulfonyl, für gegebenenfalls durch Fluor und/oder Chloratome, gegebenenfalls substituiertes zweifach verknüpftes Dioxyalkylen mit 1 bis 2 Kohlenstoffatomen, insbesondere für $OCH_2O$, $OCH_2CH_2O$, $OCF_2CF_2O$, $OCF_2O$, OCClFCClFO,

$R_5$ steht für Wasserstoff, fur Methyl oder Ethyl, die substituiert sind durch CN, durch Alkyl mit 1 bis 4 Kohlenstoffatomen, insbesondere Methyl, Alkenyl mit 2 bis 4 Kohlenstoffatomen, insbesondere $-CH=CH_2$ oder -CH=CHMe, Alkinyl mit 2 bis 4 Kohlenstoffatomen, insbesondere -CCH oder -CCMe, durch Alkoxy mit 1 bis 4 Kohlenstoffatomen, insbesondere Alkoxy wie Methoxy, Ethoxy, Propoxy, i-Propoxy, durch Alkinyloxy mit 3 bis 4 Kohlenstoffatomen, insbesondere $-OCH_2CCH$ oder $-OCH_2CCMe$, durch Alkylcarbonyl mit 1 bis 4 Kohlenstoffatomen, insbesondere Acetyl, Propionyl, i-Propionyl oder t-Butionyl, durch gegebenenfalls substituiertes Phenylcarbonyl, insbesondere Benzoyl, durch gegebenenfalls substituiertes Alkoxycarbonyl mit 1 bis 4 Kohlenstoffatomen, insbesondere $-CO_2Me$, $-CO_2Et$, $-CO_2Pr$, $-CO_2i$-Pr oder $-CO_2tBu$, durch acyliertes Amino mit 1 bis 6 Kohlenstoffatomen, wobei $R_7$ gleich Wasserstoff oder Alkyl mit 1 bis 4 Kohlenstoffatomen oder Cycloalkyl mit 3 bis 6 Kohlenstoffatomen, insbesondere $-N(Me)CO_2Me$, $-N(Me)CO_2Et$, $-N(Et)CO_2Et$, $-N(Et)CO_2Me$, - $N(Pr)CO_2Et$, $-N(Bu)CO_2Me$, $-N(t\text{-}Bu)CO_2Me$, $-N(Bu)CO_2Et$, - $N(C_6H_{11})CO_2Et$, -NHCOMe, -NHCOEt oder -NHCOPr, durch gegebenenfalls substituiertes Alkylsulfonylamino mit 1 bis 4 Kohlenstoffatomen, gegebenenfalls substituiertes Alkylsulfonyl-N-alkylamino, insbesondere $-NMeSO_2Me$, $-NEtSO_2Et$, $NMeSO_2Et$, - $NEtSO_2Me$, gegebenenfalls substituiertes Phenylsulfonylamino, gegebenenfalls substituiertes Phenylsulfonyl-N-alkylamino mit 1 bis 4 Kohlenstoffatomen, insbesondere $-NMeSO_2Ph$, $-NEtSO_2Ph$. Ferner steht $R_5$ für die gegebenenfalls substituierten Reste Alkyloxycarbonyl mit 1 bis 4 Kohlenstoffatomen, Alkylsulfonyl oder Alkenylsulfonyl mit 1 bis 4 Kohlenstoffatomen, insbesondere $MeSO_2$-, $EtSO_2$-, $PrSO_2$- oder $CH_2=CMeCH_2SO_2$-, gegebenenfalls substituiertes Phenylsulfonyl, insbesondere Phenylsulfonyl oder 2,4,6-Trimethylphenylsulfonyl, gegebenenfalls substituiertes Heteroarylsulfonyl, wobei Heteroaryl fur einen 5 oder 6-gliedrigen Heterocyclus steht, der durch Fluor, Chlor oder

Brom substituiert ist und/oder durch ein Alkyl mit 1 bis 4 Kohlenstoffatomen, insbesondere Methyl und/oder durch eine unter $R_5$ aufgefuhrten Substituenten substituiert ist, insbesondere $MeO_2C$ und ein bis drei gleiche oder verschiedene Heteroatome enthält, insbesondere Stickstoff, Schwefel und/oder Sauerstoff, Phenoxycarbonyl, oder $-SO_2NR_8R_9$, $-CONR_8R_9$, bzw. $-PO(NR_8R_9)_2$, wobei $R_8$ und $R_9$ fur Wasserstoff oder ein Alkyl mit 1 bis 4 Kohlenstoffatomen steht, die gegebenenfalls durch eine der bei $R_5$ oben genannten Reste substituiert sind, insbesondere fur $-SO_2NMe_2$ oder $-SO_2NEt_2$, $PO(NMe_2)_2$, $CONMe_2$ oder $CONiPr_2$,

$R_6$ fur $CF_3$, $CHF_2$ oder $C_2F_5$ steht.

Im einzelnen seien die folgenden substituierten Benzimidazole der allgemeinen Formel (I) genannt.

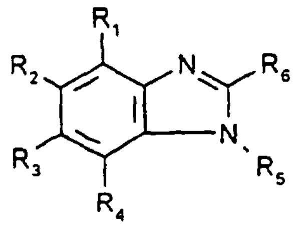

(I)

| Nr | $R_1$ | $R_2$ | $R_3$ | $R_4$ | $R_5$ | $R_6$ |
|---|---|---|---|---|---|---|
| 1 | H | Br | $CF_3$ | H | H | $CF_3$ |
| 2 | H | Br | $CF_3$ | H | $CH_2CN$ | $CF_3$ |
| 3 | H | $CF_3$ | Br | H | $CH_2CN$ | $CF_3$ |
| 4 | H | Br | $CF_3$ | H | $CH_2OEt$ | $CF_3$ |
| 5 | H | $CF_3$ | Br | H | $CH_2OEt$ | $CF_3$ |
| 6 | H | Br | $CF_3$ | H | $CH_2OiPr$ | $CF_3$ |
| 7 | H | $CF_3$ | Br | H | $CH_2OiPr$ | $CF_3$ |
| 8 | H | Br | $CF_3$ | H | $CH_2COMe$ | $CF_3$ |
| 9 | H | $CF_3$ | Br | H | $CH_2COMe$ | $CF_3$ |
| 10 | H | Br | $CF_3$ | H | $CH_2OCH(CH_2F)_2$ | $CF_3$ |
| 11 | H | $CF_3$ | Br | H | $CH_2OCH(CH_2F)_2$ | $CF_3$ |
| 12 | H | Br | $CF_3$ | H | $CH_2N(Me)CO_2Me$ | $CF_3$ |
| 13 | H | $CF_3$ | Br | H | $CH_2N(Me)CO_2Me$ | $CF_3$ |
| 14 | H | Br | $CF_3$ | H | $CH_2N(Bu)CO_2Et$ | $CF_3$ |
| 15 | H | $CF_3$ | Br | H | $CH_2N(Bu)CO_2Et$ | $CF_3$ |
| 16 | H | Br | $CF_3$ | H | $CH_2N(t\text{-}Bu)CO_2Et$ | $CF_3$ |
| 17 | H | $CF_3$ | Br | H | $CH_2N(t\text{-}Bu)CO_2Et$ | $CF_3$ |
| 18 | H | Br | $CF_3$ | H | $CH_2N(C_6H_{11})\text{-}CO_2Et$ | $CF_3$ |
| 19 | H | $CF_3$ | Br | H | $CH_2N(C_6H_{11})\text{-}CO_2Et$ | $CF_3$ |
| 20 | H | Cl | $CF_3$ | H | H | $CF_3$ |
| 21 | H | Cl | $CF_3$ | H | $CH_2CN$ | $CF_3$ |
| 22 | H | $CF_3$ | Cl | H | $CH_2CN$ | $CF_3$ |
| 23 | H | Cl | $CF_3$ | H | $CH_2OEt$ | $CF_3$ |
| 24 | H | $CF_3$ | Cl | H | $CH_2OEt$ | $CF_3$ |

| Nr | $R_1$ | $R_2$ | $R_3$ | $R_4$ | $R_5$ | $R_6$ |
|---|---|---|---|---|---|---|
| 25 | H | Cl | $CF_3$ | H | $CH_2COMe$ | $CF_3$ |
| 26 | H | $CF_3$ | Cl | H | $CH_2COMe$ | $CF_3$ |
| 27 | H | Cl | $CF_3$ | H | CH(Me)COMe | $CF_3$ |
| 28 | H | $CF_3$ | Cl | H | CH(Me)COMe | $CF_3$ |
| 29 | H | Cl | $CF_3$ | H | $CH_2COtBu$ | $CF_3$ |
| 30 | H | $CF_3$ | Cl | H | $CH_2COtBu$ | $CF_3$ |
| 31 | H | Cl | $CF_3$ | H | $CH_2COPh$ | $CF_3$ |
| 32 | H | $CF_3$ | Cl | H | $CH_2COPh$ | $CF_3$ |
| 33 | H | Cl | $CF_3$ | H | $CH_2CCH$ | $CF_3$ |
| 34 | H | $CF_3$ | Cl | H | $CH_2CCH$ | $CF_3$ |
| 35 | H | Cl | $CF_3$ | H | $CH_2NHCOMe$ | $CF_3$ |
| 36 | H | $CF_3$ | Cl | H | $CH_2NHCOMe$ | $CF_3$ |
| 37 | H | Cl | $CF_3$ | H | $CH_2N(tBu)CO_2Me$ | $CF_3$ |
| 38 | H | $CF_3$ | Cl | H | $CH_2N(tBu)CO_2Me$ | $CF_3$ |
| 39 | H | Cl | $CF_3$ | H | $CH_2N(Me)CO_2Me$ | $CF_3$ |
| 40 | H | $CF_3$ | Cl | H | $CH_2N(Me)CO_2Me$ | $CF_3$ |
| 41 | H | $CF_3$ | Cl | H | $CH_2N(Et)CO_2Me$ | $CF_3$ |
| 42 | H | Cl | $CF_3$ | H | $CH_2N(Et)CO_2Me$ | $CF_3$ |
| 43 | H | $CF_3$ | Cl | H | $CH_2N(Me)SO_2Ph$ | $CF_3$ |
| 44 | H | Cl | $CF_3$ | H | $CH_2N(Me)SO_2Ph$ | $CF_3$ |
| 45 | H | $CF_3$ | Cl | H | $CH_2N(Me)SO_2Me$ | $CF_3$ |
| 46 | H | Cl | $CF_3$ | H | $CH_2N(Me)SO_2Me$ | $CF_3$ |
| 47 | H | $SOCF_3$ | Cl | H | H | $CF_3$ |
| 48 | H | $SO_2CF_3$ | Cl | H | H | $CF_3$ |

| Nr | $R_1$ | $R_2$ | $R_3$ | $R_4$ | $R_5$ | $R_6$ |
|---|---|---|---|---|---|---|
| 49 | H | $OCF_3$ | Cl | H | H | $CF_3$ |
| 50 | H | $OCF_3$ | Cl | H | $CH_2N(Bu)CO_2Et$ | $CF_3$ |
| 51 | H | Cl | $OCF_3$ | H | $CH_2N(Bu)CO_2Et$ | $CF_3$ |
| 52 | H | $OCF_3$ | Cl | H | $CH_2N(Pr)CO_2Et$ | $CF_3$ |
| 53 | H | Cl | $OCF_3$ | H | $CH_2N(Pr)CO_2Et$ | $CF_3$ |
| 54 | H | $OCF_3$ | Cl | H | $CH_2N(Me)CO_2Me$ | $CF_3$ |
| 55 | H | Cl | $OCF_3$ | H | $CH_2N(Me)CO_2Me$ | $CF_3$ |
| 56 | H | $OCF_3$ | Cl | H | $CH_2CN$ | $CF_3$ |
| 57 | H | Cl | $OCF_3$ | H | $CH_2CN$ | $CF_3$ |
| 58 | H | Br | $OCF_3$ | H | H | $CF_3$ |
| 59 | H | Br | $OCF_3$ | H | $CH_2CN$ | $CF_3$ |
| 60 | H | $OCF_3$ | Br | H | $CH_2CN$ | $CF_3$ |
| 61 | H | Br | $OCF_3$ | H | $CH_2OEt$ | $CF_3$ |
| 62 | H | $OCF_3$ | Br | H | $CH_2OEt$ | $CF_3$ |
| 63 | H | $OCF_3$ | Br | H | $SO_2NMe_2$ | $CF_3$ |
| 64 | H | Br | $OCF_3$ | H | $CH_2N(Me)CO_2Me$ | $CF_3$ |
| 65 | H | $OCF_3$ | Br | H | $CH_2N(Me)CO_2Me$ | $CF_3$ |
| 66 | H | Br | $OCF_3$ | H | $CH_2N(Et)CO_2Et$ | $CF_3$ |
| 67 | H | $OCF_3$ | Br | H | $CH_2N(Et)CO_2Et$ | $CF_3$ |
| 68 | H | Br | $OCF_3$ | H | $CH_2N(Me)CO_2Et$ | $CF_3$ |
| 69 | H | $CF_3O$ | $OCH_3$ | H | H | $CF_3$ |
| 70 | H | $CF_3$ | $OCH_3$ | H | H | $CF_3$ |
| 71 | H | $OCF_3$ | $OCF_3$ | H | $SO_2NMe_2$ | $CF_3$ |
| 72 | H | $CF_3O$ | $OCF_3$ | H | $CH_2OCH_2CCH$ | $CF_3$ |

| Nr. | $R_1$ | $R_2$ | $R_3$ | $R_4$ | $R_5$ | $R_6$ |
|---|---|---|---|---|---|---|
| 73 | H | $OCF_2O$ | | H | H | $CF_3$ |
| 74 | H | $OCF_2CF_2O$ | | H | H | $CF_3$ |
| 75 | H | $OCF_2CF_2O$ | | H | H | $CHF_2$ |
| 76 | H | $OCF_2CF_2O$ | | H | H | $C_2F_5$ |
| 77 | H | $OCF_2CF_2O$ | | H | $CH_2CN$ | $CF_3$ |
| 78 | H | $OCF_2CF_2O$ | | H | $CH_2OEt$ | $CF_3$ |
| 79 | H | $OCF_2CF_2O$ | | H | $CH_2OiPr$ | $CF_3$ |
| 80 | H | $OCF_2CF_2O$ | | H | $CH_2N(Me)CO_2Et$ | $CF_3$ |
| 81 | H | $OCF_2CF_2O$ | | H | $CH_2N(Me)CO_2Me$ | $CF_3$ |
| 82 | H | $OCF_2CF_2O$ | | H | $CH_2N(C_6H_{11})CO_2Et$ | $CF_3$ |
| 83 | H | $OCF_2CF_2O$ | | H | $CH_2N(C_6H_{11})CO_2Et$ | $CHF_2$ |
| 84 | H | $OCFHCF_2O$ | | H | H | $CF_3$ |
| 85 | H | $O(CClF)_2O$ | | H | H | $CF_3$ |
| 86 | H | $O(CClF)_2O$ | | H | $CH_2OEt$ | $CF_3$ |
| 87 | H | $O(CClF)_2O$ | | H | $CH_2N(Me)CO_2Et$ | $CF_3$ |
| 88 | H | $O(CClF)_2O$ | | H | $CH_2CN$ | $CF_3$ |
| 89 | H | $O(CH_2)_2O$ | | H | $CH_2OEt$ | $CF_3$ |
| 90 | Cl | H | $SO_2CF_3$ | H | H | $CF_3$ |
| 91 | Cl | H | $SO_2CF_3$ | H | H | $CHF_2$ |
| 92 | Br | H | $CF_3$ | H | $CH_2COtBu$ | $CF_3$ |
| 93 | Br | H | $CF_3$ | H | $CH_2OEt$ | $CF_3$ |
| 94 | Br | H | $CF_3$ | H | $CH_2CO_2Bu$ | $CF_3$ |
| 95 | Br | H | $CF_3$ | H | $CH_2N(Me)CO_2Me$ | $CF_3$ |
| 96 | Br | H | $CF_3$ | H | $CH_2CH=CH_2$ | $CF_3$ |

| Nr | $R_1$ | $R_2$ | $R_3$ | $R_4$ | $R_5$ | $R_6$ |
|---|---|---|---|---|---|---|
| 97 | Br | H | $CF_3$ | H | H | $CF_3$ |
| 98 | Br | H | $CF_3$ | H | $CH_2N(iPr)CO_2Et$ | $CF_3$ |
| 99 | Br | H | $CF_3$ | H | $CH_2N(C_6H_{11})CO_2Et$ | $CF_3$ |
| 100 | Br | H | $SO_2Me$ | H | H | $CF_3$ |
| 101 | Br | H | $SO_2CF_2$ | H | H | $CF_3$ |
| 102 | $CF_3$ | H | Cl | H | H | $CF_3$ |
| 103 | $CF_3$ | H | Cl | H | $CH_2CN$ | $CF_3$ |
| 104 | $CF_3$ | H | Cl | H | $CH_2COPh$ | $CF_3$ |
| 105 | $CF_3$ | H | Cl | H | $CH_2OEt$ | $CF_3$ |
| 106 | $CF_3$ | H | Cl | H | $CH_2N(Me)CO_2Me$ | $CF_3$ |
| 107 | $CF_3$ | H | Cl | H | $CH_2N(Me)CO_2Et$ | $CF_3$ |
| 108 | $CF_3$ | H | Cl | H | $CH_2N(tBu)CO_2Et$ | $CF_3$ |
| 109 | $CF_3$ | H | Cl | H | $CH_2N(Bu)CO_2Et$ | $CF_3$ |
| 110 | $CF_3$ | H | Cl | H | $CH_2N(Et)CO_2Et$ | $CF_3$ |
| 111 | $CF_3$ | H | Cl | H | $CH_2N(C_6H_{11})CO_2Et$ | $CF_3$ |
| 112 | $CF_3$ | H | Br | H | H | $CF_3$ |
| 113 | $CF_3$ | H | Br | H | H | $CHF_2$ |
| 114 | $CF_3$ | H | Br | H | $CH_2CN$ | $CF_3$ |
| 115 | $CF_3$ | H | Br | H | $CH_2N(Me)SO_2Me$ | $CF_3$ |
| 116 | $CF_3$ | H | Br | H | $CH_2OPr$ | $CF_3$ |
| 117 | $CF_3$ | H | Br | H | $CH_2N(Me)CO_2Me$ | $CF_3$ |
| 118 | $CF_3$ | H | Br | H | $CH_2N(Pr)CO_2Et$ | $CF_3$ |
| 119 | $CF_3$ | H | Br | H | $CH_2N(Et)CO_2Et$ | $CF_3$ |
| 120 | $CF_3$ | H | Br | H | $CH_2N(C_6H_{11})CO_2Et$ | $CF_3$ |

| Nr. | $R_1$ | $R_2$ | $R_3$ | $R_4$ | $R_5$ | $R_6$ |
|---|---|---|---|---|---|---|
| 121 | $CF_3$ | H | $CF_3$ | H | H | $CF_3$ |
| 122 | $CF_3$ | H | $CF_3$ | H | H | $CHF_2$ |
| 123 | $CF_3$ | H | $CF_3$ | H | $CH_2CN$ | $CF_3$ |
| 124 | $CF_3$ | H | $CF_3$ | H | $CH_2N(Et)CO_2Et$ | $CF_3$ |
| 125 | $CF_3$ | H | $CF_3$ | H | $CH_2N(Pr)CO_2Et$ | $CF_3$ |
| 126 | $CF_3$ | H | $CF_3$ | H | $CH_2N(C_6H_{11})CO_2Et$ | $CF_3$ |
| 127 | Br | H | $SCF_3$ | H | H | $CF_3$ |
| 128 | Br | H | $SCF_3$ | H | $CH_2N(Pr)CO_2Et$ | $CF_3$ |
| 129 | CN | H | $CF_3$ | H | H | $CF_3$ |
| 130 | CN | H | $CF_3$ | H | H | $CHF_2$ |
| 131 | $CF_3$ | $OCF_2$-<br>$CHFCF_3$ | H | H | $SO_2NMe_2$ | $CF_3$ |
| 132 | H | $SCF_3$ | | H | H | $CF_3$ |
| 133 | H | $SCF_3$ | | | $CH_2OEt$ | $CF_3$ |
| 134 | H | $SO_2Me$ | | | H | $CF_3$ |
| 135 | H | $SO_2CF_3$ | | | H | $CF_3$ |
| 136 | H | $OCF_3$ | H | H | H | $CF_3$ |
| 137 | Br | H | $CF_3$ | H | $CON(CH_3)_2$ | $CF_3$ |
| 138 | Br | H | $CF_3$ | H | 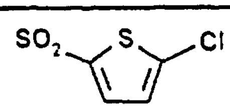 | $CF_3$ |
| 139 | Br | H | $CF_3$ | H | $SO_2CH_2C(CH_3)CH_2$ | $CF_3$ |
| 140 | Br | H | $CF_3$ | H | 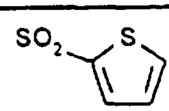 | $CF_3$ |

| Nr | $R_1$ | $R_2$ | $R_3$ | $R_4$ | $R_5$ | $R_6$ |
|---|---|---|---|---|---|---|
| 141 | Br | H | $CF_3$ | H | 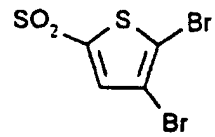 | $CF_3$ |
| 142 | Br | H | $CF_3$ | H | 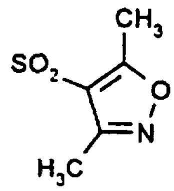 | $CF_3$ |
| 143 | Br | H | $CF_3$ | H | $COOCH(CH_3)_2$ | $CF_3$ |
| 144 | Br | H | $CF_3$ | H | $SO_2$-$CH_3$ | $CF_3$ |
| 145 | Br | H | $CF_3$ | H | $PO(N(CH_3)_2)_2$ | $CF_3$ |
| 146 | H | $OCF_2O$ | | H | 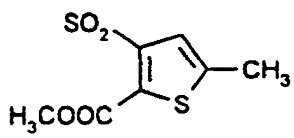 | $CF_3$ |
| 147 | H | $OCF_2O$ | | H | 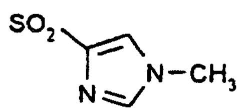 | $CF_3$ |
| 148 | H | $OCF_2OCF_2O$ | | H | 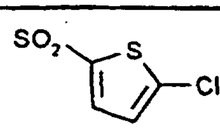 | $CF_3$ |
| 149 | H | $OCF_2OCF_2O$ | | H | $CON(C(CH_3)_2)_2$ | $CF_3$ |
| 150 | H | $OCF_2OCF_2O$ | | H | 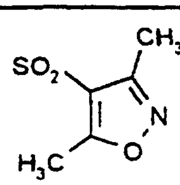 | $CF_3$ |

| Nr | $R_1$ | $R_2$ | $R_3$ | $R_4$ | $R_5$ | $R_6$ |
|---|---|---|---|---|---|---|
| 151 | H | $OCF_2OCF_2O$ | | H | 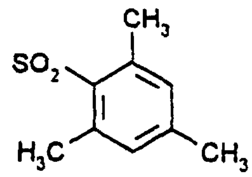 | $CF_3$ |
| 152 | H | $OCF_2O$ | | H | 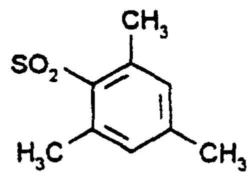 | $CF_3$ |
| 153 | H | $OCF_2OCF_2O$ | | H | 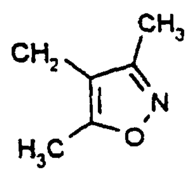 | $CF_3$ |
| 154 | H | $OCF_2O$ | | H | $SO_2CH_2C(CH_3)CH_2CF_3$ | $CF_3$ |
| 155 | H | $OCF_2O$ | | H | 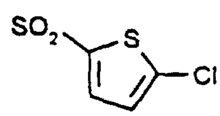 | $CF_3$ |
| 156 | H | $OCF_2OCF_2O$ | | H | 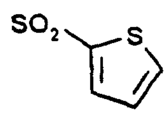 | $CF_3$ |
| 157 | H | $OCF_2O$ | | H | 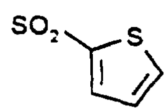 | $CF_3$ |

| Nr | $R_1$ | $R_2$ | $R_3$ | $R_4$ | $R_5$ | $R_6$ |
|---|---|---|---|---|---|---|
| 158 | H | $OCF_2OCF_2O$ | | H | 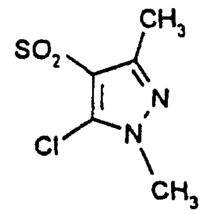 | $CF_3$ |
| 159 | H | $OCF_2O$ | | H | 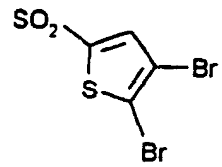 | $CF_3$ |
| 160 | H | $OCF_2CF_2O$ | | H | 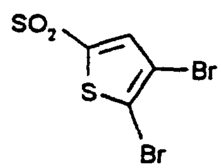 | $CF_3$ |
| 161 | H | $OCF_2O$ | | H | 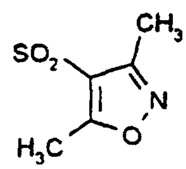 | $CF_3$ |
| 162 | H | Br | $CF_3$ | H | $SO_2CH_3$ | $CF_3$ |
| 163 | H | Br | $CF_3$ | H | 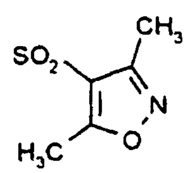 | $CF_3$ |
| 164 | H | Br | $CF_3$ | H | $SO_2N(CH_3)_2$ | $CF_3$ |
| 165 | H | $OCF_2CF_2O$ | | H | $SO_2CH_2C(CH_3)CH_2$ | $CF_3$ |
| 166 | H | $OCF_2CF_2O$ | | H | $SO_2CH_3$ | $CF_3$ |

| Nr. | $R_1$ | $R_2$ | $R_3$ | $R_4$ | $R_5$ | $R_6$ |
|---|---|---|---|---|---|---|
| 167 | H | $OCF_2CF_2O$ | | H | $PO-(N(CH_3)_2)_2$ | $CF_3$ |
| 168 | H | Br | $CF_3$ | H | $CO-OCH(CH_3)_2$ | $CF_3$ |
| 169 | H | Br | $CF_3$ | H | $CO_2N(CH_3)_2$ | $CF_3$ |
| 170 | H | Br | $CF_3$ | H | $PO-(N(CH_3)_2)_2$ | $CF_3$ |

Als besonders bevorzugte Verbindungen aus der Reihe der substituierten Benzimidazole seien genannt

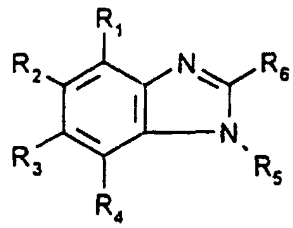

(I)

| Nr | $R_1$ | $R_2$ | $R_3$ | $R_4$ | $R_5$ | $R_6$ |
|---|---|---|---|---|---|---|
| 1 | H | Br | $CF_3$ | H | H | $CF_3$ |
| 2 | H | Br | $CF_3$ | H | $CH_2CN$ | $CF_3$ |
| 3 | H | $CF_3$ | Br | H | $CH_2CN$ | $CF_3$ |
| 4 | H | Br | $CF_3$ | H | $CH_2OEt$ | $CF_3$ |
| 5 | H | $CF_3$ | Br | H | $CH_2OEt$ | $CF_3$ |
| 58 | H | Br | $OCF_3$ | H | H | $CF_3$ |
| 59 | H | Br | $OCF_3$ | H | $CH_2CN$ | $CF_3$ |
| 60 | H | $OCF_3$ | Br | H | $CH_2CN$ | $CF_3$ |
| 74 | H | $OCF_2-CF_2O$ | | H | H | $CF_3$ |
| 83 | H | $OCF_2-CF_2O$ | | H | $CH_2N(C_6H_{11})-CO_2Et$ | $CF_3$ |
| 92 | Br | H | $CF_3$ | H | $CH_2COtBu$ | $CF_3$ |
| 95 | Br | H | $CF_3$ | H | $CH_2N(Me)CO_2Me$ | $CF_3$ |
| 96 | Br | H | $CF_3$ | H | $CH_2CH=CH_2$ | $CF_3$ |
| 99 | Br | H | $CF_3$ | H | $CH_2N(C_6H_{11})-CO_2Et$ | $CF_3$ |
| 102 | $CF_3$ | H | Cl | H | H | $CF_3$ |
| 103 | $CF_3$ | H | Cl | H | $CH_2CN$ | $CF_3$ |
| 105 | $CF_3$ | H | Cl | H | $CH_2OEt$ | $CF_3$ |
| 107 | $CF_3$ | H | Cl | H | $CH_2N(Me)CO_2Et$ | $CF_3$ |
| 108 | $CF_3$ | H | Cl | H | $CH_2N(tBu)CO_2Et$ | $CF_3$ |

(fortgesetzt)

| Nr | $R_1$ | $R_2$ | $R_3$ | $R_4$ | $R_5$ | $R_6$ |
|---|---|---|---|---|---|---|
| 112 | $CF_3$ | H | Br | H | H | $CF_3$ |
| 120 | $CF_3$ | H | Br | H | $CH_2N(C_6H_{11})$-$CO_2Et$ | $CF_3$ |

Als synthetische Coccidiosemittel bzw. als Polyetherantibiotika zur Verwendung in den erfindungsgemäßen Mischungen seien bevorzugt genannt:

Amprolium, z.T. in Kombination mit Folsäureantagonisten
Robenidin
Toltrazuril
Monensin
Salinomycin
Maduramycin

Die erfindungsgemäßen Mischungen eignen sich bei gunstiger Warmblutertoxizität zur Bekämpfung von parasitischen Protozoen, die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam Durch die Bekämpfung der parasitischen Protozoen sollen Krankheit, Todesfalle und Leistungsminderungen (z.B. bei der Produktion von Fleisch, Milch, Wolle, Hauten, Eiern, Honig usw.) vermindert werden, so daß durch den Einsatz der Wirkstoffe eine wirtschaftlichere und einfachere Tierhaltung möglich ist

Zu den parasitischen Protozoen zahlen

Mastigophora (Flagellata) wie z B Trypanosomatidae z B Trypanosoma b brucei, T.b gambiense, T b. rhodesiense, T congolense, T cruzi, T evansi, T equinum, T. lewisi, T. percae, T. simiae, T vivax, Leishmania brasiliensis, L donovani, L. tropica, wie z.B. Trichomonadidae z.B Giardia lamblia, G. canis.

Sarcomastigophora (Rhizopoda) wie Entamoebidae z.B. Entamoeba histolytica, Hartmanellidae z.B. Acanthamoeba sp , Hartmanella sp

Apicomplexa (Sporozoa) wie Eimeridae z.B Eimeria acervulina, E. adenoides, E. alabahmensis, E. anatis, E. anseris, E. arloingi, E. ashata, E. auburnensis, E. bovis, E brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana,E leucarti, E. magna, E. maxima, E media, E. meleagridis, E. meleagrimitis, E. mitis, E. necatrix, E ninakohlyakimovae, E. ovis, E. parva,E. pavonis, E. perforans, E. phasani, E. piriformis, E. praecox, E. residua, E. scabra, E. spec., E. stiedai, E. suis, E. tenella, E truncata, E. truttae, E. zuernii, Globidium spec., Isospora belli, I canis, I. felis, I. ohioensis, I. rivolta, I. spec., I. suis, Cystisospora spec., Cryptosporidium spec. wie Toxoplasmadidae z.B. Toxoplasma gondii, wie Sarcocystidae z.B Sarcocystis bovicanis, S. bovihominis, S. ovicanis, S. ovifelis, S. spec., S. suihominis wie Leucozoidae z.B. Leucozytozoon simondi, wie Plasmodiidae z.B. Plasmodium bergher, P. falciparum, P malariae, P ovale, P vivax, P. spec, wie Piroplasmea z. B. Babesia argentina, B. bovis, B. canis, B spec., Theileria parva, Theileria spec, wie Adeleina z.B Hepatozoon canis, H spec.

Zu den Nutz- und Zuchttieren gehören Saugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B Hühner, Ganse, Puten, Enten, Tauben, Vogelarten für Heim- und Zoohaltung

Zu Labor-und Versuchstieren gehoren Mäuse, Ratten, Meerschweinchen, Goldhamster, Hunde und Katzen

Zu den Hobbytieren gehoren Hunde und Katzen

Die Anwendung kann sowohl prophylaktisch als auch therapeutisch erfolgen

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Spruhens (Sprayen), Badens, Waschens, Aufgießens (pour-on and spot-on) und desEinpudems Die parenterale Anwendung geschieht z.B. in Form der Injektion (intramusculär, subcutan, intravenös,

intraperitoneal) oder durch Implantate.

Geeignete Zubereitungen sind:

Lösungen wie Injektionslosungen, orale Lösungen, Konzentrate zur oralen Verabreichung nach Verdunnung, Lösungen zum Gebrauch auf der Haut oder in Körperhöhlen, Aufgußformulierungen, Gele,

Emulsionen und Suspension zur oralen oder dermalen Anwendung sowie zur Injektion; Halbfeste Zubereitungen;

Formulierungen, bei denen der Wirkstoff in einer Salbengrundlage oder in einer Öl in Wasser oder Wasser in Öl Emulsionsgrundlage verarbeitet ist,

Feste Zubereitungen wie Pulver, Premixe oder Konzentrate, Granulate, Pellets, Tabletten, Boli, Kapseln; Aerosole und Inhalate, wirkstoffhaltige Formkorper.

Injektionslösungen werden intravenos, intramuskulär und subcutan verabreicht

Injektionslosungen werden hergestellt, indem der Wirkstoff in einem geeigneten Losungsmittel gelost wird und eventuell Zusatze wie Losungsvermittler, Sauren, Basen, Puffersalze, Antioxidantien, Konservierungsmittel zugefugt werden Die Losungen werden steril filtriert und abgefüllt

Als Losungsmittel seien genannt. Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben

Die Wirkstoffe lassen sich gegebenenfalls auch in physiologisch verträglichen pflanzlichen oder synthetischen Ölen, die zur Injektion geeignet sind, lösen.

Als Losungsvermittler seien genannt: Lösungsmittel, die die Losung des Wirkstoffs im Hauptlösungsmittel fördern oder sein Ausfallen verhindern Beispiele sind Polyvinylpyrrolidon, polyoxyethyliertes Rhizinusol, polyoxyethylierte Sorbitanester.

Konservierungsmittel sind: Benzylalkohol, Trichlorbutanol, p-Hydroxybenzoesaureester, n-Butanol.

Orale Lösungen werden direkt angewendet. Konzentrate werden nach vorheriger Verdunnung auf die Anwendungskonzentration oral angewendet. Orale Lösungen und Konzentrate werden, wie oben bei den Injektionslösungen beschrieben, hergestellt, wobei auf steriles Arbeiten verzichtet werden kann.

Lösungen zum Gebrauch auf der Haut werden aufgetraufelt, aufgestrichen, eingerieben, aufgespritzt, aufgesprüht oder durch Tauchen (Dippen), Baden oder Waschen aufgebracht. Diese Lösungen werden, wie oben bei den Injektionslosungen beschrieben, hergestellt.

Es kann vorteilhaft sein, bei der Herstellung Verdickungsmittel zuzufugen Verdickungsmittel sind. Anorganische Verdickungsmittel wie Bentonite, kolloidale Kieselsaure, Aluminiummonostearat, organische Verdickungsmittel wie Cellulosederivate, Polyvinylalkohole und deren Copolymere, Acrylate und Metacrylate

Gele werden auf die aufgetragen oder aufgestrichen oder in Körperhöhlen eingebracht Gele werden hergestellt, indem Losungen, die wie bei den Injektionslosungen beschrieben hergestellt worden sind, mit soviel Verdikkungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautvertraglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Losungsmittel seien genannt: Wasser, Alkanole,Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Ole, DMF, Dimethylacetamid, N-Methylpyrrolidon, 2-Dimethyl-4-oxy-methylen-1,3-dioxolan

Farbstoffe sind alle zur Anwendung am Tier zugelassenen Farbstoffe, die gelost oder suspendiert sein können.

Resorptionsfördernde Stoffe sind z.B. DMSO, spreitende Ole wie Isopropylmyristat, Dipropylenglykolpelargonat, Silikonöle, Fettsaureester, Triglyceride. Fettalkohole.

Antioxidantien sind Sulfite oder Metabisulfite wie Kaliummetabisulfit, Ascorbinsaure, Butylhydroxytoluol, Butylhydroxyanisol, Tocopherol

Lichtschutzmittel sind z B Stoffe aus der Klasse der Benzophenone oder Novantisolsäure

Haftmittel sind z B Cellulosederivate, Stärkederivate, Polyacrylate, naturliche Polymere wie Alginate, Gelatine

Emulsionen konnen oral, dermal oder als Injektionen angewendet werden.

Emulsionen sind entweder vom Typ Wasser in Ol oder von Typ Ol in Wasser

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert

Als hydrophobe Phase (Ole) seien genannt: Paraffinole, Silikonöle, natürliche Pflanzenöle wie Sesamöl, Mandelöl, Rizinusöl, synthetische Triglyceride wie Capryl/Caprinsaure-biglycerid, Triglyceridgemisch mit Pflanzenfettsäuren der Kettenlänge $C_{8-12}$ oder anderen speziell ausgewählten natürlichen Fettsauren, Partialglycendgemische gesättigter oder ungesattigter, eventuell auch hydroxylgruppenhaltiger Fettsäuren, Mono-und Diglyceride der $C_8/C_{10}$-Fettsäuren

Fettsaureester wie Ethylstearat, Di-n-butyryl-adipat, Laurinsäurehexylester, Dipropylen-glykolpelargonat, Ester einer verzweigten Fettsaure mittlerer Kettenlänge mit gesättigten Fettalkoholen der Kettenlange $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Olsäureoleylester,Ölsauredecylester, Ethyloleat, Milchsäureethylester, wachsartige Fettsäureester wie Dibutylphthalat, Adipinsaurediisopropylester, letzterem verwandte Estergemische u a Fettalkohole wie Isotridecylalkohol, 2-Octyldodecanol, Cetylstearylalkohol, Oleylalkohol

Fettsauren wie z B. Olsäure und ihre Gemische

Als hydrophile Phase seien genannt.
Wasser, Alkohole wie z B. Propylenglycol, Glycenn, Sorbitol und ihre Gemische.

Als Emulgatoren seien genannt:
nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz; kationaktive Tenside wie Cetyltrimethylammoniumchlorid.

Als weitere Hilfsstoffe seien genannt:

Viskositatserhohende und die Emulsion stabilisierende Stoffe wie Carboxymethylcellulose, Methylcellulose und andere Cellulose-und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsaure oder Gemische der aufgeführten Stoffe

Suspensionen konnen oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfordernde Stoffe, Konservierungsstoffe, Antioxidantien, Lichtschutzmittel suspendiert

Als Trägerflüssigkeiten seien alle homogenen Losungsmittel und Lösungsmittelgemische genannt

Als Netzmittel (Dispergiermittel) seien die weiter oben angegebenen Tenside genannt.

Als weitere Hilfsstoffe seien die weiter oben angegebenen genannt.
Halbfeste Zubereitungen können oral oder dermal verabreicht werden. Sie unterscheiden sich von den oben beschriebenen Suspensionen und Emulsionen nur durch ihre hohere Viskositat.

Zur Herstellung fester Zubereitungen wird der Wirkstoff mit geeigneten Tragerstoffen gegebenenfalls unter Zusatz von Hilfsstoffen vermischt und in die gewunschte Form gebracht.

Als Tragerstoffe seien genannt alle physiologisch vertraglichen festen Inertstoffe Alle solche dienen anorganische und organische Stoffe. Anorganische Stoffe sind z B Kochsalz, Carbonate wie Calciumcarbonat, Hydrogencarbonate, Aluminiumoxide, Kieselsauren, Tonerden, gefälltes oder kolloidales Siliciumdioxid, Phosphate

Organische Stoffe sind z.B. Zucker, Zellulose, Nahrungs-und Futtermittel wie Milchpulver, Tiermehle, Getreidemehle und -schrote, Stärken

Hilfsstoffe sind Konservierungsstoffe, Antioxidantien, Farbstoffe, die bereits weiter oben aufgeführt worden sind

Weitere geeignete Hilfsstoffe sind Schmier-und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen

Anwendungsfertige Zubereitungen enthalten die Wirkstoffe in Konzentrationen von 10 ppm bis 20 Gewichtsprozent, bevorzugt von 0,1 bis 10 Gewichtsprozent

Die Benzimidazole der Formel (I) liegen dabei in folgendem Gewichtsverhältnis an den synthetischen Coccidiosemittein bzw. Polyetherantibiotika vor 1 zu 0,1 - 10, bevorzugt 1 zu 1 - 10

Zubereitungen die vor Anwendung verdunnt werden, enthalten die Wirkstoffe in Konzentrationen von 0,5 bis 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoffe je kg Körpergewicht pro Tag zur Erzielung wirksamer Ergebnisse zu verabreichen.

Die Wirkstoffe können auch zusammen mit dem Futter oder Trinkwasser der Tiere verabreicht werden

Futter-und Nahrungsmittel enthalten 0,01 bis 250 ppm, vorzugsweise 0.5 bis 100 ppm des Wirkstoffs in Kombination mit einem geeigneten eßbaren Material

Ein solches Futter-und Nahrungsmittel kann sowohl für Heilzwecke als auch für prophylaktische Zwecke verwendet werden.

Die Herstellung eines solchen Futter-oder Nahrungsmittels erfolgt durch Mischen eines Konzentrats oder einer Vormischung, die 0,5 bis 30 %, vorzugsweise 1 bis 20 Gew -% eines Wirkstoffs in Mischung mit einem eßbaren organischen oder anorganischen Träger enthält mit üblichen Futtermitteln Eßbare Träger sind z.B. Maismehl oder Mais-und Sojabohnenmehl oder Mineralsalze, die vorzugsweise eine geringe Menge eines eßbaren Staubverhütungsöls, z.B Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Coccidiose genannt:

Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt. Falls gewunscht, konnen diese Mengen erhöht werden, besonders wenn der Wirkstoff vom Empfänger gut vertragen wird Entsprechend kann die Verabreichung über das Trinkwasser erfolgen

Für die Behandlung von Einzeltieren, z.B. im Falle der Behandlung der Coccidiose bei Säugetieren oder der Toxoplasmose, werden vorzugsweise Wirkstoffmengen von 0,5 bis 100 mg/kg Korpergewicht taglich verabreicht, um die gewünschten Ergebnisse zu erzielen Trotzdem kann es zeitweilig notwendig sein, von den genannten Mengen abzuweichen, insbesondere in Abhangigkeit vom Korpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genugen, mit weniger als der vorstehend genannten Mindestmenge auszukommen, wahrend in anderen Fallen die genannte obere Grenze uberschritten werden muß Bei der Verabreichung großerer Mengen kann es zweckmaßig sein, diese im Verlauf des Tages in mehrere Einzeldarreichungen zu unterteilen

Die Wirksamkeit der erfindungsgemäßen Mischungen läßt sich z.B. in Käfigversuchen mit folgender Versuchsanordnung belegen, bei der die Tiere mit den jeweiligen Einzelkomponenten sowie mit den Mischungen der Einzelkomponenten behandelt werden.

Käfigversuch Coccidiose/Küken

Coccidienfrei aufgezogene 8 bis 12 Tage alte männliche Hühnerküken (z.B. LSL Brinkschulte/Senden) erhalten von 3 Tagen vor (Tag -3) der Infektion (= a.i.) bis 8 (9) Tage nach der Infektion (= p.i.) die erfindungsgemaßen Verbindungen (Testsubstanzen) in der in ppm angegebenen Konzentration mit dem Futter. In jedem Käfig werden 3 Tiere gehalten. Je Dosierung werden ein bis mehrere derartige Gruppen eingesetzt. Die Infektion erfolgt mittels einer Schlundsonde direkt in den Kropf mit etwa 50 000 sporulierten Oocysten von Eimeria acervulina sowie mit jeweils etwa 20 000 sporulierten Oocysten von E maxima und E. tenella. Es handelt sich hierbei um hochvirulente Stämme. Die genaue Infektionsdosis wird so eingestellt, daß möglichst eins von drei experimentell infizierten unbehandelten Küken infektionsbedingt stirbt. Für die Beurteilung der Wirksamkeit werden die folgenden Kriterien berücksichtigt Gewichtszunahme von Versuchsbeginn bis Versuchsende, Infektionsbedingte Sterberate, makroskopische Beurteilung der Faeces hinsichtlich Durchfall und Blutausscheidung an den Tagen 5 und 7 p.i. (Bewertung 0 bis 6), makroskopische Beurteilung der Darmschleimhaut, insbesondere der Blinddarme (Bewertung 0 bis 6) und die Oocystenausscheidung sowie der Anteil (in %) der innerhalb von 24 Stunden sporulierenden Oocysten Die Zahl der Oocysten im Kot wurde mit Hilfe der McMaster-Zahlkammer bestimmt (siehe Engelbrecht und Mitarbeiter "Parasitologische Arbeitsmethoden in Medizin und Veterinarmedizin, Akademie-Verlag, Berlin (1965)). Die einzelnen Befunde werden in Relation zu den unbehandelten nicht infizierten Kontroll-Gruppen gesetzt und eine Gesamtbewertung errechnet (vgtl A. Haberkorn (1986) S 263 bis 270 in Research in Avtan Coccidiosis ed L.R McDougald, L.P Joyner, P L Long Proceedings of the Georgia Coccidiosis Conference Nov, 18 - 20 1985 Athens/Georgia USA)

Ein wirkstoffhaltiges Futter wird so zubereitet, daß die erforderliche Menge Wirkstoff mit einem nährstoffmäßig ausgeglichenen Tierfutter, z.B. mit dem in dem unten angegebenen Kükenfutter, grundlich vermischt wird

Wenn ein Konzentrat oder eine Vormischung zubereitet werden soll, die schließlich im Futter auf die im Versuch genannten Werte verdünnt werden soll, werden im allgemeinen etwa 1 bis 30 %, vorzugsweise etwa 10 bis 20 Gewichtsprozent Wirkstoff mit einem eßbaren organischen oder anorganischen Träger, z.B. Mais- und Sojamehl oder Mineralsalzen, die eine kleine Menge eines eßbaren Entstaubungsöls, z.B. Maisöl oder Sojabohnenöl enthalten, vermischt. Die so erhaltene Vormischung kann dann dem vollstandigen Geflügelfutter vor der Verabreichung zugegeben werden.

Als Beispiel fur die Verwendung der erfindungsgemäßen Stoffe im Geflügelfutter kommt die folgende Zusammensetzung in Frage.

| | |
|---|---|
| 52,00 % | Futtergetreideschrot, und zwar 40 % Mais, 12 % Weizen |
| 17,00 % | Sojaschrot extr. |
| 5,00 % | Maiskleberfutter |
| 5,00 % | Weizenfuttermehl |
| 3,00 % | Fischmehl |
| 3,00 % | Mineralstoffmischung |
| 3,00 % | Luzernegrasgrunmehl |
| 2,50 % | Vitaminvormischung |
| 2,00 % | Weizenkeime, zerkleinert |
| 2,00 % | Sojaol |
| 2,00 % | Fleischknochenmehl |
| 1,50 % | Molkenpulver |
| 1.00 % | Melasse |
| 1,00 % | Bierhefe, gebunden an Biertreber |
| 100,00 % | |

Ein solches Futter enthält 18 % Rohprotein, 5 % Rohfaser, 1 % Ca, 0,7 % P sowie je kg 1 200 i.E Vitamin A, 1 200 i.E Vitamin $D_3$, 10 mg Vitamin E, 20 mg Zinkbacitracin.

Versuchsergebnisse mit erfindungsgemaßen Kombinationen sind in den folgenden Tabellen beispielhaft aufgeführt. Die synergistische Wirksamkeit der Kombinationen im Vergleich zu den Einzelkomponenten wird besonders an der Reduktion der Oocystenausscheidung aber auch bezüglich der Sektionsbefunde, Gewichtsentwicklung und besseren Verträglichkeit ersichtlich.

In den folgenden Tabellen bedeutet in Spalte "Treatment" die Angabe

| | |
|---|---|
| n.inf.contr | = nicht infizierte Kontrollgruppe |
| inf.contr | = infizierte Kontrollgruppe |
| Amprol Komb | = Amprolium kombiniert mit Sulfaquinoxalin und Ethopabat |
| 1 | = Benzimidazol Bsp. Nr. |

In der Spalte "ppm" wird die eingesetzte Konzentration des Wirkstoffs im Futter in ppm angegeben.

In der Spalte "mortality" wird angegeben unter % der Prozentsatz der gestorbenen Tiere und unter n die Anzahl der gestorbenen Tiere/im Versuch eingesetzten Tiere.

In der Spalte "weight % of not inf control" wird das Verhaltnis des Gewichts der behandelten Tiere zum Gewicht der nicht infizierten Kontrollgruppe angegeben

In den Spalten "dropping scores", "lesion Score" und "oocyst control" werden Einzelangaben zur Wirkung gemacht

In der Spalte "% efficay" wird die Gesamtwertung bonitiert. 0 % bedeutet keine Wirkung, 100 % bedeutet volle Wirkung

Tabelle 1

| Experimentelle Infektion mit Eimeria acervulina, E. maxima and E. tenella an Kuken. | | | | | | | | | | | |
|---|---|---|---|---|---|---|---|---|---|---|---|
| Treatment | ppm | mortality | | weight % of not inf. control | dropping cores | lesion score | oocyst in % of inf control | | | | % efficay tot |
| | | % | n | | | | ac | max. | ten. | tot. | |
| n. inf contr | 0 | 0 | 0/6 | 100 | 0 | 0 | 0 | 0 | 0 | 0 | 100 |
| inf contr. | 0 | 50 | 3/6 | 46 | 6 | 6 | 100<br>915* | 100<br>55* | 100<br>1520* | 100<br>2490* | 0 |
| Amprol Komb. | 30 | 0 | 0/3 | 71 | | 0,7 | 63 | 36 | 46 | 52 | 36 |
| | 75 | 0 | 0/3 | 75 | | 0 | 100 | 36 | 100 | 78 | 42 |
| 1 | 5 | 0 | 0/3 | 80 | 4-6 | 4 | 15 | 100 | 14 | 43 | 39 |
| Amprol. Komp + 1 | 50 + 5 | 0 | 0/3 | 89 | 0 | 0 | 1.5 | 2 | 1 | 1.5 | 82 |
| | 75 +5 | 0 | 0/3 | 95 | 0 | 0,7 | 0,26 | 0 | 0,32 | 0.29 | 98 |

* = x 1 000

Tabelle 2

| Treatment | ppm | mortality | | weight % of not inf. control | dropping scores | lesion score | oocyst in % of inf control | | | | % efficay tot |
|---|---|---|---|---|---|---|---|---|---|---|---|
| | | % | n | | | | ac. | max. | ten | tot. | |
| n. inf contr | 0 | 0 | 0/6 | 100 | 0 | 0 | 0 | 0 | 0 | 0 | 100 |
| inf contr | 0 | 0 | 0/6 | 61 | | 6 | 100<br>3810* | 100<br>480* | 100<br>2980* | 100<br>7270* | 0 |
| Monensin | 25 | 0 | 0/3 | 44 | | 6 | 44 | 100 | 100 | 81 | 7 |
| | 50 | 0 | 0/3 | 88 | | 6 | 13 | 33 | 19 | 22 | 43 |
| 1 | 2,5 | 33 | 1/3 | 83 | 6 | 6 | 14 | 38 | 41 | 31 | 35 |
| | 5 | 0 | 0/3 | 76 | 6 | 5,7 | 0,6 | 3 | 3 | 2,1 | 69 |
| | 10 | 33 | 1/3# | 100 | 0-2 | 3,5 | 0 | 0 | 0 | 0 | 92 |
| Monensin + 1 | 25 2.55 | 0 | 0/3 | 66 | 6 | 6 | 13 | 29 | 24 | 22 | 32 |
| | 25 + 5 | 0 | 0/3 | 102 | 0-2 | 4,3 | <0,1 | <0,1 | 0.1 | 0.1 | 92 |
| | 25 + 10 | 0 | 0/3 | 102 | 0-2 | 0.7 | 0 | 0 | <0,1 | <0,1 | 98 |
| | 50 + 2.5 | 0 | 0/3 | 97 | 1 | 0.3 | <0.1 | 0.1 | <0.1 | <0.1 | 96 |

* = x 1 000

# = wegen Toxizitat

Tabelle 3

| Treatment | ppm | mortality | | weight % of not inf control | dropping scores | lesion score | oocyst in % of inf. control | | | | % efficay tot |
|---|---|---|---|---|---|---|---|---|---|---|---|
| | | % | n | | | | ac | max | ten | tot. | |
| n inf. contr | 0 | 0 | 0/6 | 100 | 0 | 0 | 0 | 0 | 0 | 0 | 100 |
| inf contr. | 0 | 83 | 5/6 | 64 | 6 | 6 | 100<br>1420* | 100<br>220* | 100<br>3560* | 100<br>5200 * | 0 |
| Robenidin | 16,5 | 0 | 0/3 | 95 | 1 | 5 | 38 | 9 | 23 | 23 | 60 |
| 1 | 5 | 67 | 2/3 | 87 | 6 | 6 | 11 | 15 | 11 | 12 | 40 |
| | 10 | 33 | 1/3# | 83 | 6 | 6 | 0,8 | 0.9 | 0,6 | 0.8 | 71 |
| Robenidin + 1 | 16.5 + 5 | 0 | 0/3 | 114 | 0 | 1,3 | 1,2 | 4 | 2 | 9 | 88 |
| | 16.5 + 10 | 33 | 1/3'# | 96 | 0 | 4 | 0,01 | 0 | 0 | <0.01 | 94 |

* = x 1 000

# = wegen Toxizitat

Tabelle 4

| Treatment | ppm | mortality | | weight % of not inf control | dropping scores | lesion score | oocyst in % of inf control | | | | % efficay tot. |
|---|---|---|---|---|---|---|---|---|---|---|---|
| | | % | n | | | | ac. | max | ten. | tot | |
| n. inf. contr. | 0 | 0 | 0/6 | 100 | 0 | 0 | 0 | 0 | 0 | 0 | 100 |
| inf. contr. | 0 | 83 | 5/6 | 64 | 6 | 6 | 100<br>1430* | 100<br>220* | 100<br>3560* | 100<br>5200* | 0 |
| Toltrazuril | 10 | 0 | 0/3 | 84 | 3 | 6 | 3 | 4 | 1 | 3 | 68 |
| | 15 | 0 | 0/3 | 106 | 5 | 3,3 | 0,6 | 0 | 0,4 | 0,3 | 88 |
| 1 | 5 | 67 | 2/3 | 87 | 6 | 6 | 11 | 15 | 11 | 12 | 40 |
| | 10 | 33 | 1/3# | 83 | 6 | 6 | 0.8 | 0,9 | 0,6 | 0,8 | 71 |
| Toltrazuril + 1 | 10 + 5 | 0 | 0/3 | 76 | 6 | 6 | 0,7 | 2 | 0,6 | 1,1 | 72 |
| | 15 + 10 | 0 | 0/3 | 94 | 0 | 4,3 | 0 | 0 | 0 | 0 | 98 |

* = x 1 000

# = wegen Vergiftung

Tabelle 5

| Treatment | ppm | mortality | | weight % of not inf. control | dropping scores | lesion score | oocyst in % of int control | | | | % efficay tot |
|---|---|---|---|---|---|---|---|---|---|---|---|
| | | % | n | | | | ac | max | ten | tot. | |
| n. inf contr | 0 | 0 | 0/6 | 100 | 0 | 0 | 0 | 0 | 0 | 0 | 100 |
| inf contr | 0 | 0 | 0,6 | 62 | 6 | 6 | 100<br>1260* | 100<br>150* | 100<br>1640* | 100<br>3050* | 0 |
| Monensin | 25 | 0 | 0/3 | 72 | 4 | 5,3 | 86 | >100 | >100 | 95 | 0 |
| | 50 | 0 | 0/3 | 78 | 6 | 6 | 35 | 93 | 38 | 55 | 30 |
| | 100 | 0 | 0/3 | 92 | 0 | 2,7 | 11 | 7 | 20 | 13 | 69 |
| 74 | 5 | 0 | 0/3 | 59 | 6 | 6 | >100 | 40 | >100 | 80 | 4.3 |
| | 10 | 33 | 1/3# | 80 | 0 | 4,5 | 0.7 | 3 | 2 | 1,9 | 73 |
| Monensin + 74 | 25+ 5 | 0 | 0/3 | 83 | 0 | 5,3 | 8 | 9 | 15 | 11 | 58 |
| | 25 + 10 | 0 | 0/3 | 80 | 4 | 4.3 | 1 | 0 | 0.9 | 0.6 | 69 |
| | 50 + 5 | 0 | 0/3 | 90 | 0 | 2.3 | 0.08 | 0 | 0.13 | 0.07 | 98 |
| | 50 + 10 | 33 | 1/3# | 98 | 0 | 0,5 | 0.7 | 0 | 0,05 | 0,25 | 100 |
| | 100 + 5 | 0 | 0/3 | 89 | 0 | 1,3 | 1 | 0.7 | 4 | 1,9 | 85 |
| | 100 + 5 | 0 | 0/3 | 87 | 0 | 0,7 | 0 | 0 | 0,02 | 0,01 | 87 |

* = x 1 000

\# = wegen Vergiftung

Tabelle 6

| Treatment | ppm | mortality | | weight % of not inf. control | dropping scores | lesion score | oocyst in % of inf. control | | | | % efficay tot |
|---|---|---|---|---|---|---|---|---|---|---|---|
| | | % | n | | | | ac | max. | ten. | tot. | |
| n. inf contr. | 0 | 0 | 0/6 | 100 | 0 | 0 | 0 | 0 | 0 | 0 | 100 |
| inf contr. | 0 | 0 | 0/6 | 59 | 6 | 6 | 100<br>2760* | 100<br>200* | 100<br>1820* | 100<br>4780* | 0 |
| Halofuginon | 1 | 0 | 0/3 | 67 | 2 | 2 | 50 | 50 | 96 | 66 | 26 |
| | 3 | 0 | 0/3 | 72 | 0 | 0,7 | 5 | 4 | 19 | 9 | 70 |
| 74 | 5 | 0 | 0/3 | 38 | 6 | 6 | 84 | 100 | 45 | 76 | 4 |
| | 10 | 33 | 1/3 | 48 | 6 | 6 | 100 | 44 | 44 | 63 | 7 |
| Halofuginon 74 | 1 + 10 | 0 | 0/3 | 55 | 0 | 0,7 | 2,5 | 6 | 13 | 7,2 | 53 |
| | 3 + 5 | 0 | 0/3 | 76 | 0 | 0 | 3 | 0 | 5 | 2,7 | 85 |

* = x 1 000

Tabelle 7

| Treatment | ppm | mortality | | weight % of not inf control | droppmg scores | lesion score | oocyst in % of inf. control | | | | % efficay tot |
|---|---|---|---|---|---|---|---|---|---|---|---|
| | | % | n | | | | ac | max. | ten | tot | |
| n int. contr | 0 | 0 | 0/6 | 100 | 0 | 0 | 0 | 0 | 0 | 0 | 100 |
| inf contr | 0 | 33 | 2/6 | 62 | 6 | 6 | 100<br>645* | 100<br>115* | 100<br>865* | 100<br>1625* | 0 |
| Salinomycin | 15 | 0 | 0/3 | 85 | 6 | 6 | 81 | 87 | 58 | 75 | 16 |
| | 30 | 0 | 0/3 | 100 | 3·5 | 2,3 | 9 | 0 | 22 | 10 | 74 |
| | 40 | 0 | 0/3 | 112 | 0 | 0 | 2,5 | 5 | 1,7 | 3 | 90 |
| 60 | 10 | 0 | 0/3 | 65 | 6 | 6 | 59 | 70 | 53 | 61 | 5 |
| | 12.5 | 0 | 0/3 | 86 | 6 | 6 | 71 | 100 | 65 | 79 | 16 |
| | 15 | 0 | 0/3 | 85 | 6 | 6 | 62 | 100 | 72 | 78 | 16 |
| | 20 | 0 | 0/3 | 84 | 4-6 | 2,3 | 31 | 87 | 40 | 53 | 32 |
| Salinomycin+ 60 | 15 + 10 | 0 | 0/3 | 100 | 6 | 2,3 | 28 | 70 | 53 | 50 | 37 |
| | 15 + 12.5 | 0 | 0/3 | 100 | 0 | 0,3 | 0,09 | 0,5 | 0.2 | 0.27 | 100 |
| | 30 + 10 | 0 | 0/3 | 98 | 0 | 0,3 | 0 | 0 | 0 | 0 | 100 |
| | 30 + 12.5 | 0 | 0/3 | 96 | 0 | 0 | 0 | 0.2 | 0 | 0,07 | 100 |
| | 30 + 15 | 0 | 0/3 | 103 | 1 | 1 | 0 | 0 | 0 | 0 | 96 |
| | 30 + 20 | 0 | 0/3 | 101 | 0 | 0.7 | 0 | 0 | 0 | 0 | 98 |
| | 40 + 10 | 0 | 0/3 | 100 | 0 | 0 | 0.03 | 0 | 0.1 | 0,03 | 100 |
| | 40 + 12.5 | 0 | 0/3 | 94 | 2 | 0.7 | 0 | 0 | 0 | 0 | 95 |
| | 40 + 20 | 0 | 0/3 | 97 | 0 | 0 | 0 | 0 | 0 | 0 | 100 |

* = x 1 000

Tabelle 8

| Treatment | ppm | mortality | | weight % of not inf control | dropping scores | lesion score | oocyst in % of inf control | | | | % efficay tot |
|---|---|---|---|---|---|---|---|---|---|---|---|
| | | % | n | | | | ac. | max | ten. | tot | |
| n. inf contr | 0 | 0 | 0/6 | 100 | 0 | 0 | 0 | 0 | 0 | 0 | 100 |
| inf contr. | 0 | 17 | 1/6 | 61 | 6 | 6 | 100<br>1080* | 100<br>220* | 100<br>2550* | 100<br>3850* | 0 |
| Toltrazuril | 2,5 | 0 | 0/3 | 59 | 6 | 5,3 | 12 | 9 | 11 | 11 | 23 |
| 59 | 5 | 0 | 0/3 | 61 | 6 | 6 | 91 | 100 | 99 | 100 | 0 |
| | 25 | 33 | 1/3# | 84 | 1 | 5 | 0 | 0 | 0,2 | 0,1 | 79 |
| 59 + Toltrazuril | 2,5 + 5 | 0 | 0/3 | 69 | 6 | 5.7 | 11 | 9 | 12 | 11 | 33 |
| | 2.5 + 25 | 0 | 0/3 | 92 | 0 | 2 | 0 | 0 | 0 | 0 | 100 |

* = x 1 000

\# = wegen Toxizität

Tabelle 9

| Treatment | ppm | mortality | | weight % of not inf control | dropping scores | lesion score | oocyst in % of inf control | | | | % efficay tot |
|---|---|---|---|---|---|---|---|---|---|---|---|
| | | % | n | | | | ac. | max. | ten | tot. | |
| n inf. contr | 0 | 0 | 0/6 | 100 | 0 | 0 | 0 | 0 | 0 | 0 | 100 |
| inf contr. | 0 | 0 | 0/6 | 54 | 6 | 6 | 100<br>1570* | 100<br>70* | 100<br>1057* | 100<br>2697* | 0 |
| Halofuginon | 1 | 33 | 1/3 | 81 | 4-6 | 6 | 41 | 100 | 49 | 63 | 24 |
| | 3 | 0 | 0/3 | 87 | 0 | 0 | <0.1 | 0 | <0.1 | <0.1 | 91 |
| 112 | 1 | 0 | 0/3 | 41 | 6 | 6 | 100 | 57 | 91 | 83 | 0 |
| | 5 | 0 | 0/3 | 88 | 0-2 | 6 | 0.8 | 10 | 2,4 | 4,4 | 77 |
| Halofuginon 112 | 1 + 1 | 33 | 1/3 | 88 | 6 | 6 | 12 | 100 | 17 | 43 | 30 |
| | 3+ 1 | 0 | 0/3 | 102 | 0 | 0 | 1 | 0 | 0,7 | 0,6 | 96 |
| | 3 + 5 | 0 | 0/3 | 83 | 0 | 1,7 | <0.1 | 06 | 0.2 | 0,1 | 89 |

* = x 1 000

## Patentansprüche

1. Mischungen von substituierten Benzimidazolen der Formel (I)

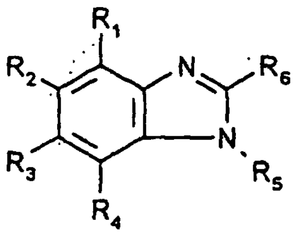

(I)

in welcher

$R_1$, $R_2$, $R_3$ und $R_4$ unabhängig voneinander jeweils für Wasserstoff, Halogen, für jeweils gegebenenfalls substituiertes Alkyl, Alkoxy, Alkylthio, Alkylsulfinyl, Alkylsulfonyl, für gegebenenfalls substituiertes ankondensiertes Dioxyalkylen stehen, wobei jedoch mindestens einer der Substituenten $R_1$, $R_2$, $R_3$ und $R_4$ verschieden von Wasserstoff und Halogen ist,

$R_5$ für Wasserstoff, für Alkyl steht, das ein- oder mehrfach, gleich oder verschieden substituiert ist durch OH, CN, $NH_2$, Alkyl, Cycloalkyl, Alkenyl, Alkinyl, Alkoxy, Halogenalkoxy, Alkylthio, Halogenalkylthio, Alkenoxy, Alkinoxy, Aminocarbonyl, gegebenenfalls substituiertes Alkylcarbonyl, gegebenenfalls substituiertes (Het-)arylcarbonyl, gegebenenfalls substituiertes Alkoxycarbonyl (AlkO-CO-), gegebenenfalls substituiertes Alkoxycarbonyloxy (AlkOCOO-), Aminosulfonyl ($SO_2NH_2$), gegebenenfalls substituiertes Mono- oder Dialkylaminosulfonyl, acyliertes Amino (AlkCON($R_7$)- oder AlkOCON($R_7$)-), wobei $R_7$ gleich Wasserstoff, Alkyl oder Cycloalkyl ist, gegebenenfalls substituiertes Alkylsulfonylamino (Alkyl-$SO_2$NH-), oder Alkylsulfonyl-N-alkylamino (Aryl$SO_2$NAlkyl-), gegebenenfalls substituiertes Arylsulfonylamino (Aryl$SO_2$NH-) oder Arylsulfonyl-N-alkylamino (Aryl$SO_2$NAlk-), gegebenenfalls substituiertes Dialkylamino, ferner steht $R_5$ für gegebenenfalls substituiertes Alkoxycarbonyl, gegebenenfalls substituiertes (Het-)Aryloxycarbonyl, Alkylsulfonyl, Alkenylsulfonyl, (Het-)Arylsulfonyl, oder $-SO_2NR_8R_9$, $-CONR_8R_9$ bzw. $-P(O)(NR_8R_9)_2$, wobei $R_8$ und $R_9$ für H oder Alkyl stehen, das gegebenenfalls durch einen oder mehrere Reste substituiert ist,

$R_6$ für Fluoralkyl steht,

mit einer oder mehreren der folgenden Verbindungen:
Polyetherantibiotika wie Maduramycin, Lasalocid, Monensin, Narasin, Salinomycin oder synthetische Coccidiosemittel ausgewählt aus

| | |
|---|---|
| 1(-(4-Amino-2-n-propyl-5-pyrimidinylmethyl)-2-picolinium-chlorid | Amprolium |
| 1(-(4-Amino-2-n-propyl-5-pyrimidinylmethyl)-2-picolinium-chlorid + Sulfaquinoxalin | Amprolium + Sulfaquinoxalin |
| 1(-(4-Amino-2-n-propyl-5-pyrimidinylmethyl)-2-picolinium-chlorid + Sulfaquinoxalin + Ethopabate | Amprolium + Sulfaquinoxalin + Ethopabate |
| 4,4-Dinitrocarbanilid + 2-Hydroxy-4,6-dimethylpyrimidin | Nicarbazin |
| 3,5-Dichloro-2,6-dimethyl-4-pyridinol | Clopidol |
| 3,5-Dichloro-2,6-dimethyl-4-pyridinol + methyl-7-benzyloxy-6-butyl-1,4-dihydro-4-oxylquinolin-3-carboxylate | Clopidol + Methylbenzoquate |
| Ethyl 6-n-decyloxy-7-ethoxy-4-hydroxychinolin-3-carboxylat | Decoquinate |
| 9-(2-Chloro-6-fluorophenylmethyl)-9H-purin-6-amin | Arprinocid |
| (±)-2,6-Dichloro-alpha-(4-chlorophenyl)-4-(4,5-dihydro-3,5-dioxo-1,2,4-triazin-2(3H)-yl)-benzeneacetonitril | Benzeneacetonitril, Diclazuril |

(fortgesetzt)

| | |
|---|---|
| 1-[3-Methyl-4-(4'-trifluoromethylthiophenoxy)-phenyl]-3-methyl-1,3,5-triazin-2,4,6(1H,3H,5H)-trion | Toltrazuril |
| 4,4-Dinitrocarbanilid + 2-Hydroxy-4,6-dimethylpyrimidine [= Nicarbazin] | Robenidin |
| 7-Bromo-6-chloro-febrifugin | Halofuginon |
| 3,5-Dinitro-o-toluamid | Zoalen |

als Mittel zur Bekämpfung parasitärer Protozoen, insbesondere Coccidien bei Tieren.

2. Verwendung von Mischungen von Benzimidazolen der Formel (I) und Polyetherantibiotika und synthetischen Coccidiosemitteln gemäß Anspruch 1 zur Herstellung von Mitteln gegen parasitäre Protozoen bei Tieren.

3. Verwendung von Mischungen gemäß Anspruch 1 zur Herstellung von Mittelen zur Bekämpfung von Coccidien bei Tieren.

## Claims

1. Mixtures of substituted benzimidazoles of the formula (I)

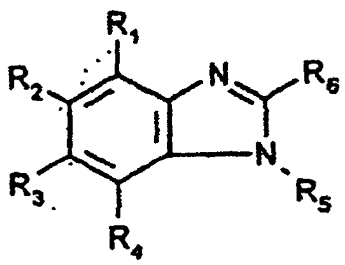

(I)

in which

| | |
|---|---|
| $R_1$, $R_2$, $R_3$ and $R_4$ | each, independently of one another, represent hydrogen, halogen, represent in each case optionally substituted alkyl, alkoxy, alkylthio, alkylsulphinyl, alkylsulphonyl, represent optionally substituted fused-on dioxyalkylene, but where at least one of the substituents $R_1$, $R_2$, $R_3$ and $R_4$ is different from hydrogen and halogen, |
| $R_5$ | represents hydrogen, represents alkyl which is substituted one or more times, identically or differently, by OH, CN, $NH_2$, alkyl, cycloalkyl, alkenyl, alkinyl, alkoxy, halogenoalkoxy, alkylthio, halogenoalkylthio, alkenoxy, alkinoxy, aminocarbonyl, optionally substituted alkylcarbonyl, optionally substituted (het-)arylcarbonyl, optionally substituted alkoxycarbonyl (AlkO-CO-), optionally substituted alkoxycarbonyloxy (AlkOCOO-), aminosulphonyl ($SO_2NH_2$), optionally substituted mono- or dialkylaminosulphonyl, acylated amino (AlkCON($R_7$)- or AlkOCON($R_7$)-), where $R_7$ is equal to hydrogen, alkyl or cycloalkyl, or optionally substituted alkylsulphonylamino (Alkyl$SO_2$NH-), or alkylsulphonyl-N-alkylamino (Aryl$SO_2$NAlkyl-), optionally substituted arylsulphonylamino (Aryl$SO_2$NH-) or arylsulphonyl-N-alkylamino (Aryl$SO_2$NAlk-), optionally substituted dialkylamino, furthermore $R_5$ represents optionally substituted alkoxycarbonyl, optionally substituted (het-)aryloxycarbonyl, alkylsulphonyl, alkenylsulphonyl, (het-)arylsulphonyl, or $-SO_2NR_8R_9$, $-CONR_8R_9$ or - P(O)($NR_8R_9$)$_2$, where $R_8$ and $R_9$ represent H or alkyl which is optionally substituted by one or more radicals, |
| $R_6$ | represents fluoroalkyl, |

with one or more of the following compounds:
Polyether antibiotics such as maduramycin, lasalocid, monensin, narasin, salinomycin or synthetic coccidiostats selected from

| | |
|---|---|
| 1-(4-Amino-2-n-propyl-5-pyrimidinylmethyl)-2-picolinium chloride | Amprolium |
| 1-(4-Amino-2-n-propyl-5-pyrimidinylmethyl)-2-picolinium chloride + sulfaquinoxaline | Amprolium + sulfaquinoxaline |
| 1-(4-Amino-2-n-propyl-5-pyrimidinylmethyl)-2-picolinium chloride + sulfaquinoxaline + ethopabate | Amprolium + sulfaquinoxaline + ethopabate |
| 4,4-Dinitrocarbanilide + 2-hydroxy-4,6-dimethylpyrimidine | Nicarbazin |
| 3,5-Dichloro-2,6-dimethyl-4-pyridinol | Clopidol |
| 3,5-Dichloro-2,6-dimethyl-4-pyridinol + methyl 7-benzyloxy-6-butyl-1,4-dihydro-4-oxyquinoline-3-carboxylate | Clopidol + methylbenzoquate |
| Ethyl 6-n-decyloxy-7-ethoxy-4-hydroxyquinoline-3-carboxylate | Decoquinate |
| 9-(2-Chloro-6-fluorophenylmethyl)-9H-purin-6-amine | Arprinocid |
| (±)-2,6-Dichloro-alpha-(4-chlorophenyl)-4-(4,5-dihydro-3,5-dioxo-1,2,4-triazin-2(3H)-yl)-benzeneacetonitrile | Benzeneacetonitrile, diclazuril |
| 1-[3-Methyl-4-(4'-trifluoromethylthiophenoxy)-phenyl]-3-methyl-1,3,5-triazine-2,4,6(1H,3H,5H)-trione | Toltrazuril |
| 4,4-Dinitrocarbanilide + 2-hydroxy-4,6-dimethylpyrimidine [= nicarbazin] | Robenidine |
| 7-Bromo-6-chloro-febrifugin | Halofuginone |
| 3,5-Dinitro-o-toluamide | Zoalene |

as compositions for controlling parasitic protozoa, in particular coccidia, in livestock.

**2.** Use of mixtures of benzimidazoles of the formula (I) and polyether antibiotics and synthetic coccidiostats according to Claim 1 for the preparation of compositions to combat parasitic protozoa in livestock.

**3.** Use of mixtures according to Claim 1 for the preparation of compositions for controlling coccidia in livestock.

## Revendications

**1.** Mélanges de benzimidazoles substituées de la formule (I) :

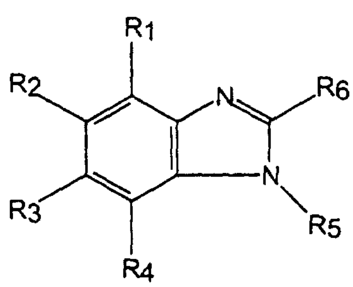

(I)

dans laquelle :

Ri, $R_2$, $R_3$ et $R_4$ représentent indépendamment l'un de l'autre, chaque fois un atome d'hydrogène, d'halogène, représentent un radical alcoyle, alcoxy, alcoylthio, alcoylsulfinyle, alcoylsulfonyle, le cas échéant substitué, représentent un radical dioxyalcoylène condensé le cas échéant substitué, où cependant, au moins un des substituants $R_1$, $R_2$, $R_3$ et $R_4$ est différent d'un atome d'hydrogène ou d'halogène ;
$R_5$ représente l'atome d'hydrogène, représente un radical alcoyle, qui est substitué une ou plusieurs fois, de manière identique ou différente, par un radical OH, CN, $NH_2$, alcoyle, cycloalcoyle, alcényle, alcynyle, alcoxy,

halogénoalcoxy, alcoylthio, halogénoalcoylthio, alcényloxy, alcynyloxy, aminocarbonyle, alcoylcarbonyle le cas échéant substitué, (het)arylcarbonyle le cas échéant substitué, alcoxycarbonyle (AlcO-CO-) .le cas échéant substitué, alcoxycarbonyloxy (AlcO-COO-) le cas échéant substitué, aminosulfonyle ($SO_2NH_2$), mono- ou dialcoylaminosulfonyle le cas échéant substitué, amino acylé (AlcCON($R_7$)- ou AlcOCON($R_7$)-), ou $R_7$ est chaque fois, l'atome d'hydrogène, un radical alcoyle ou cycloalcoyle, représente un radical alcoylsulfonylamino (Alcoyl$SO_2$NH-) le cas échéant substitué, ou alcoylsulfonyl-N-alcoylamino (Alcoyl$SO_2$Nalcoyl-) , arylsulfonylamino (Aryl$SO_2$NH-) le cas échéant substitué, ou arylsulfonul-N-alcoylamino (Aryl$SO_2$Nalc-), dialcoylamino le cas échéant substitué, de plus $R_5$ représente alcoxycarbonyle le cas échéant substitué, (het)aryloxycarbonyle le cas échéant substitué, alcoylsulfonyle, alcénylsulfonyle, (het) arylsulfonyle ou -$SO_2NR_8R_9$, -$CONR_8R_9$ ou -P(O)($NR_8R_9$)$_2$, où $R_8$ et $R_9$ représentent l'atome H ou un radical alcoyle, qui est substitué le cas échéant par un ou plusieurs restes ;

$R_6$ représente un radical fluoroalcoyle, avec un ou plusieurs des composés suivants :

des polyétherantibiotiques comme la maduramycine, le lasalocid, la monensine, la narasine, la salinomycine ou des agents synthétiques contre la coccidiose, choisis parmi :

| | |
|---|---|
| le chlorure de 1(-(4-amino-2-n-propyl-5-pyrimidinylméthyl)-2-picolinium | l'amprolium |
| le chlorure de 1(-(4-amino-2-n-propyl-5-pyrimidinylméthyl)-2-picolinium + la sulfaquinoxaline | l'amprolium + la sulfaquinoxaline |
| le chlorure de 1(-(4-amino-2-n-propyl-5-pyrimidinylméthyl)-2-picolinium + la sulfaquinoxaline + l'éthopabate | l'amprolium + la sulfaquinoxaline + l'éthopabate |
| le 4,4-dinitrocarbanilide + la 2-hydroxy-4,6-diméthylpyrimidine | le nicarbazin |
| le 3,5-dichloro-2,6-diméthyl-4-pyridinol | le clopidol |
| le 3,5-dichloro-2,6-diméthyl-4-pyridinol + le 7-benzyloxy-6-butyl-1,4-dihydro-4-méthyle | le clopidol + le méthylbenzoquate |
| le 6-n-décyloxy-7-éthoxy-4-hydroxyquinoléine-3-carboxylate d'éthyle | le décoquinate |
| la 9-(2-chloro-6-fluorophénylméthyl)-9H-purine-6-amine | l'arprinocide |
| le (±)-2,6-dichloro-alpha-(4-chlorophényl)-4-(4,5-dihydro-3,5-dioxo-1,2,4-triazine-2(3H)-yl)benzèneacétonitrile | un benzèneacétonitrile, le diclazurile |
| la 1-[3-méthyl-4-(4'-trifluorométhylthiophénoxy)phényl]-3-méthyl-1,3,5-triazine-2,4-6(1H,3H,5H)trione | la toltrazurile |
| le 4,4-dinitrocarbanilide + la 2-hydroxy-4,6-diméthylpyrimidine (= nicarbazin) | la robénidine |
| la 7-bromo-6-chlorofébrifugine | l'halofuginone |
| le 3,5-dinitro-o-toluamide | le zoalène |

comme agent pour lutter contre les protozoaires parasites, en particulier les coccidies des animaux.

**2.** Utilisation de mélanges de benzimidazoles de la formule (I) et de polyétherantibiotiques et d'agents synthétiques contre la coccidiose suivant la revendication 1, pour lutter contre les protozoaires parasites chez les animaux.

**3.** Utilisation des mélanges suivant la revendication 1, pour la préparation d'agents pour lutter contre les coccidies des animaux.